# DEMANDE DE BREVET EUROPEEN

(11) **EP 3 603 650 A1**
(43) Date de publication de la demande: **05.02.2020**
(21) Numéro de dépôt: 18306051.6
(22) Date de dépôt: 01.08.2018
(51) Int. Cl.: A61K 31/765, A61K 31/137, A61K 31/4418, A61P 31/10, A61P 33/00, A61P 31/12, A61F 2/00, A61K 9/00

(54) **COMPOSITIONS INJECTABLES ET A DUREE D'ACTION PROLONGEE POUR LEUR UTILISATION DANS LE TRAITEMENT DE MALADIES DE L'ONGLE ET/OU POUR ACCELERER LA CROISSANCE DE L'ONGLE**

(71) Demandeur: EDIX O SARL, 1331 Luxembourg (LU)
(72) Inventeur: CHERIF-CHEIKH, Roland, 08860 CASTELLDEFELS-BARCELONA (ES); LACOMBE, Frederic, 08173 SANT CUGAT DEL VALLES, BARCELONA (ES); LACHAMP, Laurence, 08850 GAVA BARCELONA (ES); CAMPRODON, Xavier Julia, 08006 BARCELONA (ES); DOMENECH AGUERA, Albert, 08014 BARCELONA (ES); SERRANO CARRENO, Marta, 08830 SANT BOI DE LLOBREGAT BARCELONA (ES)
(74) Mandataire: Bourgouin, André

(57) **Abrégé**

La présente invention a pour objet une composition pour son utilisation dans le traitement de maladies de l'ongle et/ou pour accélérer la croissance de l'ongle caractérisée en ce qu'elle comprend un ou plusieurs polymères solides biodégradables à libération prolongée, et éventuellement un ou plusieurs principes actifs et en ce qu'elle se présente sous la forme d'un dispositif destiné à être administré par voie sous-cutanée à une distance supérieure à 1cm du bord de l'ongle de préférence dans le doigt à l'exception de la dernière phalange.

## Description

La présente demande a pour objet des compositions injectables à durée d'action prolongée pour leur utilisation dans le traitement de maladies de l'ongle et/ou pour accélérer la croissance de l'ongle.

### ETAT DE LA TECHNIQUE :

Parmi les différentes maladies qui peuvent affecter les ongles ou les extrémités, on peut citer les maladies inflammatoires telles que le psoriasis, ou le lichen plan; les tumeurs de l'ongle telles que les tumeurs glomiques, les tumeurs myxomateuses, les kystes cutanés myxoïdes; les infections telles que les paronychies, les panaris, les mycoses et autres affections fongiques telles que l'onychomycose aussi appelée mycose des ongles ou mycose unguéale, l'onychatrophie, la leuconychie, l'onychogryphose, la koilonychie, la melanonychie unguéale, l'hyperkératose sous unguéale, l'onychorrhexie, la paronychie, les maladies d'origine traumatique chimique, infectieuse, inflammatoire ou autres comme la pelade, la maladie de Darrier et d'autres maladies communes connues de l'homme de l'art, non limitées aux mycoses fongiques.

Les mycoses et autres infections fongiques peuvent également affecter la peau, les muqueuses et les cheveux.

L'onychomycose est l'affection la plus fréquente de l'ongle, elle touche de 6 à 9 % de la population générale. Elle est rare chez l'enfant et sa fréquence augmente avec l'âge, elle affecte ainsi 30 % des plus de 70 ans. Aux Etats- Unis seulement on estime la prévalence à plus de 35 millions de personnes.

Au niveau mondial, 15% de la population générale souffrira au moins d'une mycose de l'ongle au cours de sa vie. Il est estimé que 50% des personnes de 70 ans ou plus en sont atteintes et du fait de leur mobilité souvent réduite et de leur polymédication, ces personnes ne sont pas convenablement traitées aujourd'hui.

Il existe de nombreux facteurs augmentant la propension à être atteint d'onychomycose : une possible prédisposition génétique, les microtraumatismes, des maladies associées (psoriasis, diabète, mauvaise circulation sanguine des pieds, immunodépression ...), certains traitements médicamenteux (immunosuppresseurs, chimiothérapies, corticoïdes...), des facteurs liés à l'âge, et surtout des facteurs environnementaux (chaleur et humidité, transpiration importante, port continu de chaussures fermées, marche pieds nus sur les sols publics, entre autres).

L'onychomycose est une infection fongique du lit unguéal, de la matrice ou de la tablette unguéale. La première des caractéristiques cliniques de l'onychomycose est l'onycholyse distale (séparation de la tablette unguéale du lit unguéal), l'hyperkératose sous-unguéale et un ongle dystrophique décoloré.

Les patients atteints d'onychomycose sont habituellement embarrassés par les imperfections de leur(s) ongle(s) et un des impacts importants reconnus de la pathologie est l'impact social ainsi que la perte de confiance en soi.

Les symptômes de la maladie sont initialement seulement esthétiques avec l'apparition de taches sur l'ongle, mais si la maladie n'est pas traitée, l'infection peut s'étendre et être accompagnée de symptômes physiques, pouvant parfois limiter la mobilité avec une difficulté lors de la marche (voire une perte de dextérité) et peuvent être associés à des douleurs. La maladie peut également diminuer indirectement la circulation périphérique et entraîner une stase veineuse, ce qui peut aggraver des états pathologiques tels que des ulcères chez les patients diabétiques.

Des infections fongiques de l'ongle peuvent également s'étendre à d'autres parties du corps et potentiellement à d'autres personnes.

Dans le cas de la mycose de l'ongle ou onychomycose, différentes espèces de champignons pathogènes peuvent être recensées. Les plus fréquentes et dans plus de 70% des cas sont des Dermatophytes, en particulier Trichophyton rubrum, Trichophyton interdigitale ou Trichophyton mentagrophytes.

Les 30% restants sont des levures du type Candida en général les Candida parapsilosis, Candida guilliermondii, Candida Albicans, ou des moisissures non dermatophytes comme les espèces Aspergillus spp, Scopulariosis spp, brevicaulis spp, Fusarium spp, Acremonium spp et Neoscytalidium spp.

On peut citer communément d'autres pathogènes dermatophytes comme Epidermophyton floccosum, Trichophyton violaceum, Microsporum gypseum, Trichophyton tonsurans ou Trichophyton souadenense.

Il existe cinq sous-types d'onychomycoses recensés et associés au degré de gravité. L'onychomycose sous-unguéale distale (DLSO) représente toutefois plus de 90% des cas recensés.

Même si les pathologies les plus courantes sont les onychomycoses, l'objet de l'invention permet de traiter également l'ensemble des mycoses mais aussi les maladies de l'ongle (onychopathies) comme celles indiquées ci-dessus et en particulier le psoriasis ou le panaris, ainsi que d'améliorer les altérations de la morphologie de l'ongle (onychodystrophies), liées ou non aux pathologies précédemment citées. L'objet de l'invention peut aussi traiter de façon générale les maladies dites des extrémités connues par l'homme de l'art.

Dans le cas de ces infections, les traitements médicamenteux actuels les plus couramment utilisés consistent en l'administration quotidienne sur plusieurs mois, voire une année entière, de formes pharmaceutiques topiques et/ou de formes orales pour l'éradication des champignons dans le cas des dermatomycoses et la récupération esthétique des zones affectées.

Il est probable et souvent avéré que pour que le traitement soit définitif ou pour éviter toutes récidives, il soit nécessaire de traiter pendant plus d'un an. Cela n'est toutefois pas envisageable en pratique avec les traitements d'applications ou de prises quotidiennes actuels.

Les antifongiques utilisés pour traiter les mycose de l'ongles peuvent de préférence comprendre la Nystatine, l'Amphotéricine B, l'Abafungine, le Benzalkonium chloride, la Caspofungine, le Cetrimide, le Clioquinol, le Sulphate de cuivre, l'Haloprogine, les Echinocandines, la Flucytosine, le Mycobactovir, la Novexatine, la Natamycine, le Cetylpyridium chlorure, la Benzylamine butenafine, les Benzoxaboroles (dérivés cycliques des acides boroniques), l'Albaconazole, l'Arasertaconazole, le Bifonazole, le Butoconazole, le Clotrimazole, l'Eberconazole, l'Éconazole, l'Eficonazole, le Fenticonazole, le Fluconazole, le Fosravuconazole, l'Isoconazole, l'Irtemazole, l'Isavuconazole, le Ketoconazole, le Liarozole, le Lianozole, le Luliconazole, le Miconazole, l'Oxadiazole, l'Oxiconazole, le Posaconazole, le Pramiconazole, le Ravuconazole, le Sertaconazole, le Sulconazole, le Terconazole, le Thiazole, le Thiabendazole, le Thiadiazole, le Thiamazole, le Tioconazole, le Voriconazole, l'Amiodarone, la Naftifine, le Tavaborole, la Butenafine, la Flucytosine, la Griséofulvine, la Caspofungine, la Micafungine, l'Oxide nitrique, l'Oxychlorosène de sodium, la Povidoneiodine, le Thiocarbonate tolnafate, la Sulbentine, le Zync Pyrithione, et plus particulièrement parmi la Terbinafine, le Ciclopirox, la Ciclopiroxolamine, l'Itraconazole, et l'Amorolfine.

Les traitements par voie orale peuvent contenir, par exemple le kétoconazole, le fluconazole, l'isavuconazole, l'itraconazole, le posaconazole ou le voriconazole; les échinocandines telles que l'anidulafungine, la caspofungine, la micafungine, la flucytosine, la griséofulvine et la terbinafine; l'amphotéricine B et en particulier le ketoconazole, l'itraconazole, la terbinafine chlorhydrate, le fluconazole la griseofulvine, et en cas de traitement d'urgence hospitalier le posaconazole ou posaconazol et le voriconazole. Ces antifongiques peuvent également être utilisés pour la mise en oeuvre de la présente invention. Toutefois, ces antifongiques oraux sont associés à des effets systémiques légers (migraines, éruptions cutanées, perte de goût ou photosensitivité), ou sérieux comme des problèmes cardiaques ou hépatiques qui limitent fortement leurs utilisations.

Des thérapies topiques avec des antifongiques tels que le fluconazole, le ketoconazole, le miconazole, la terbinafine, le tolnaftate, le sertaconazole, l'eberconazole, le fenticonazole, 1 oxiconzale, le clotrimazole, le bifonazole, sont une alternative pour les patients chez lesquels une thérapie antifongique orale est contre-indiquée.

Tous ces antifongiques peuvent également être utilisés pour la mise en oeuvre de la présente invention.

Pour les traitements des mycoses cutanées locales d'accès facile, des formes médicamenteuses telles que des crèmes, des gels, des pommades, des sprays, poudres, aérosols et lotions sont utilisés. Des vernis contenant un ou plusieurs antifongiques constituent également un traitement souvent utilisé, bien que possédant de nombreux effets secondaires, tels que : irritations, démangeaisons, éruptions cutanées, inflammation, dermatites de contact, brulures de la peau proche de la zone d'administration, ainsi que des altérations de l'ongle (ongle décoloré, ongle fragile ou ongle cassant).

Ces formes médicamenteuses d'application facile sur la zone infectée permettent d'accéder directement aux champignons et, grâce à l'effet fongistatique et fongicide des agents thérapeutiques incorporés dans les préparations, de contrôler l'infection et de l'éradiquer après des périodes de traitements pouvant durer de quelques semaines à plusieurs mois.

La mycose de l'ongle, de par sa difficulté d'accès est aujourd'hui encore l'une des plus difficiles à traiter. En effet, les champignons peuvent coloniser l'ongle au départ de son lit et se propager petit à petit, affectant la structure kératinisée de l'ongle. Si l'infection n'est pas arrêtée à temps, elle peut aller jusqu'à coloniser la totalité de l'ongle jusqu'à la matrice, avec des conséquences systémiques graves pour les patients, par exemple pour des patients diabétiques.

Le cas de mycoses avancées où la matrice est infectée, qui est un des premiers focus de l'invention, implique généralement souvent l'utilisation de traitements oraux, les traitements topiques et autres traitements du type LASER ne pénétrant pas bien jusqu'à la base de l'infection, et donc ne peuvent pas soigner à cet endroit spécifique du renouvellement de l'ongle.

De ce fait, au niveau de l'ongle, il est difficile d'accéder au champignon en utilisant des traitements classiques topiques. C'est pourquoi des systèmes ont été développés pour favoriser le traitement direct de l'ongle et faciliter le passage au travers de l'ongle des agents thérapeutiques.

Une des solutions thérapeutiques envisagées sont les vernis thérapeutiques décrits par exemple dans la demande internationale WO 2010/086723. Ils contiennent des agents filmogènes et des solvants qui s'évaporent après l'application laissant le principe actif déposé sur l'ongle.

Par exemple, la demande internationale WO 2007/147052 décrit une composition comprenant notamment de la terbinafine, pour une utilisation dans le traitement de l'onychomycose par application topique. La composition est constituée d'un vecteur de libération comprenant un inhibiteur de cristallisation non polymère, un agent filmogène et un solvant volatil.

La demande internationale WO 2011/79234 décrit des compositions topiques, antifongiques comprenant un agent antifongique, un tensioactif zwittérionique ou son dérivé présentant une charge, un acide carboxylique, un alcool de faible poids moléculaire, et de l'eau.

On peut aussi citer le cas du vernis Penlac® de la société Valeant Pharmaceuticals (solution de ciclopirox à 8%) qui a été approuvé par la Food and Drug Administration aux Etats Unis pour le traitement topique d'onychomycoses légères à modérées.

Ces vernis doivent être appliqués très régulièrement et généralement contiennent au maximum 10% d'agent thérapeutique (excepté pour le produit Trosyd® de Pfizer, pouvant aller jusqu'à 28% de tioconazole). Ces médicaments proposent un traitement local en surface des mycoses de l'ongle, mais ne s'attaquent pas au nid de l'infection. Par ailleurs, la perméation à travers l'ongle est très faible et seulement une partie infime de la dose arrive directement jusqu'à la zone à traiter.

Il est important aussi de considérer l'impact environnemental et l'utilisation de quantités importantes de solvants organiques. Plus récemment, afin de pallier cet inconvénient, de nouvelles compositions filmogènes réduisant les quantités de solvant organiques ont vu le jour ; toutefois, elles ne changent pas la posologie ni la durée du traitement.

Des efforts substantiels en recherche ont été mis sur le développement de système augmentant la perméation transunguéale des agents thérapeutiques.

Plus récemment, s'inspirant des systèmes transdermiques, des formulations pouvant être administrées par ionophorèse ont été développées, comme par exemple décrits dans la demande WO2008/121709.

Pour maximiser le contact du principe actif avec l'infection, il existe aussi des propositions impliquant un perçage de l'ongle en de multiples endroits au moyen d'un rayon LASER et un dépôt de formulations contenant les agents thérapeutiques.

De tels procédés sont décrits dans la demande de brevet international WO 02/11764 et plus récemment dans la demande WO2011/071137. Ces approches très invasives et douloureuses sont aussi coûteuses. Elles offrent l'avantage de cibler l'infection sous l'ongle mais ne s'attaquent pas par exemple aux possibles infections collatérales dues au caractère contagieux des infections fongiques.

Les onychomycoses sont en général associées également à des mycoses des zones adjacentes comme les doigts, orteils, pieds et mains. Par ailleurs, ce type d'administration, à cause de la taille des orifices faits sur l'ongle, ne permet que l'apport d'une quantité infinitésimale de principe actif qui ne présente pas d'effet retard susceptible de traiter l'ongle sur plusieurs semaines ou plusieurs mois sans renouvellement du produit.

Au niveau de la matrice, il n'est pas possible d'envisager un traitement topique satisfaisant qui agisse efficacement en profondeur dans les tissus vivants. Lorsque l'infection est présente à ce niveau, la matrice va continuer à produire un ongle malade, infecté. Dans ces cas avancés de mycoses, il faut malgré leurs effets secondaires, envisager des médicaments en prises orales qu'il peut être préférable de combiner avec les formes topiques.

Comme indiqué ci-dessus, à cause des limitations venant du manque d'accessibilité, d'efficacité, de fréquence et de durée de traitement, les médecins n'ont à ce jour pas d'autres options que d'avoir recours à des alternatives de traitement systémique par voie orale.

Ces formes orales sous forme de comprimés sont généralement administrées à haute dose quotidiennement pendant des durées limitées à 3 mois, seules ou en association avec des formes topiques contenant des agents antifongiques appliquées tous les jours.

Ces traitements oraux entraînent une forte exposition de tout l'organisme pendant une longue durée et présentent l'inconvénient d'entraîner d'importants effets secondaires, notamment de toxicité hépatique limitant leur utilisation en routine dans les cas les plus avancés de la maladie.

Dans tous les cas, et quels que soient les traitements, il faut attendre la repousse complète de l'ongle (6 mois pour la main et 12 mois pour les orteils) pour pouvoir obtenir une rémission totale ; cela impose une discipline extrême de suivi quotidien sur toute la durée des traitements. Par rémission, s'entend l'absence d'infection et le retour à une apparence esthétique normale.

Ainsi, il n'existe pas aujourd'hui de médicaments d'utilisation facile pour traiter l'onychomycose ou les maladies de l'ongle. Les traitements actuels ne permettent pas, en général, d'obtenir la guérison, car dépendante du respect strict du traitement quotidien sur une durée annuelle ou de la limite de prises des traitements avec risques d'effets secondaires. Les patients arrêtent souvent d'eux même leur traitement dès que l'aspect esthétique invalidant semble avoir disparu ou s'est atténué alors que, dans ces cas-là, il reste souvent des colonies de champignons qui se reproduisent alors rapidement et conduisent à une rechute et récidive de l'infection (absence de cure mycologique).

Comme indiqué ci-dessus, une des raisons de l'échec des traitements actuels est le manque d'observance ou compliance, c'est à dire le non-suivi par le patient des prescriptions de son médecin dans la prise de ces traitements médicamenteux et les règles hygiéno-diététiques qui y sont associées. Les traitements sont contraignants, et parfois complexes. En effet, il est par exemple très compliqué pour les patients âgés à la mobilité réduite d'utiliser quotidiennement des formes topiques difficiles à appliquer sur les ongles des pieds infectés.

L'observance est un élément clé du succès d'une thérapie médicamenteuse et plus particulièrement dans ces traitements pour lesquels il est important d'avoir en permanence la présence des produits antifongiques au niveau des sites infectés.

Cette non observance est reconnue comme une des causes principales d'échec de ces traitements.

Une autre raison de ces échecs est liée aux limites dans le temps de prises des formes orales seules capables d'assurer l'absence d'infection à la naissance de l'ongle au niveau de la matrice. De plus, les formes orales sont les seuls traitements systémiques des dermatophytoses ou autres infections cutanées, du cheveu ou des ongles. Par ailleurs, les antifongiques systémiques sont utilisés lorsque le germe en cause - un champignon microscopique - se montre résistant ou lorsqu'un traitement local est impossible.

En traitement hospitalier d'urgence et face à des infections fongiques graves comme l'Aspergillose, ou les candidoses cutanéo-muqueuses et dans les mycoses chez les patients immunodéprimés, le personnel soignant dispose de traitements antifongiques injectables, par voie intraveineuse. A titre d'exemple, il existe des solutions intraveineuses d'amphotericine B, de fluconazole, de voriconazole, d'itraconazole ou de posaconazole.

Il existe déjà des traitements connus par injections au niveau de l'extrémité des doigts et autour de l'ongle. Le traitement du psoriasis peut par exemple conduire à des injections de glucocorticoïdes anti-inflammatoires dans les lésions. Par exemple, lors de l'administration de triamcinolone dans le pli proximal de l'ongle, le long du pli latéral et de chaque côté, dans la matrice ou dans le lit de l'ongle implique des anesthésies locales car ces zones sont connues comme étant très sensibles. Ce genre de traitement entraîne souvent des effets secondaires tels que l'atrophie de l'ongle ou des hémorragies sous-unguéales.

Des demandes de brevets visant à traiter les maladies de l'ongle et en particulier l'onychomycose à l'aide de systèmes biodégradables de libération de médicaments formulés en vue d'être implantés dans l'ongle et ses tissus environnants pour le traitement de divers états pathologiques de l'ongle ont été déposées.

Un système de ce type est décrit dans la demande WO2006/086888 (équivalente à la demande américaine US2008/0299165).

Les systèmes incluent des compositions qui peuvent être aussi formulées sous forme de solides, notamment dans le cas des demandes internationales ultérieures WO 2007/139804 et WO 2011/086723.

Cependant, l'implantation d'une formulation solidifiable ou solide sous l'ongle ou dans les tissus environnants en cas de maladie et notamment en cas d'onychomycose présente de nombreux inconvénients et peut s'avérer très douloureuse.

En effet, il n'y a pas suffisamment d'espace dans ces zones spécifiques pour administrer une composition ayant un volume suffisant pour un traitement sur plusieurs mois. Il n'est pas non plus souhaitable de réaliser un geste invasif d'injection dans ou proche de la zone malade ou infectée qui va ajouter un traumatisme, voire favoriser la diffusion de l'infection.

Ce sont des zones particulièrement innervées et vascularisées ou trop proches d'une articulation, dans lesquelles l'insertion d'une aiguille et l'administration d'une forme solidifiable ou solide posent des problèmes de lésion de traumatisme et de cicatrisation voire peuvent déclencher d'autres pathologies liées par exemple à l'arthrose, qui ne sont pas favorables à la guérison du tissu infecté, qui constitue l'objectif du traitement.

Il n'est pas non plus possible de multiplier le nombre de formes solidifiables ou solides injectées dans ces zones ni de répéter aisément ces injections.

En effet, en présence de pathologies combinées, différents principes actifs et notamment différents antifongiques sont souvent nécessaires pour le traitement de ces pathologies et l'espace restreint se trouvant sous l'ongle ou dans les tissus environnants ne permet pas l'injection de ces traitements associant plusieurs principes actifs en quantité suffisante.

En effet, il est aujourd'hui reconnu qu'une des raisons supplémentaires de l'échec des traitements des maladies de l'ongle est qu'elles sont souvent causées par différents types de pathogènes et présentent souvent des conséquences associées comme l'inflammation ou autres maladies.

A titre d'exemple non limitatif, certains champignons sont parfois résistants à la terbinafine et d'autres au cyclopirox. Or, comme indiqué ci-dessus, il n'existe pas aujourd'hui pour le patient de moyens pour traiter de façon simultanée et spécifique ces mycoses sans exposer l'organisme à des doses élevées de principes actifs thérapeutiques présentant une certaine toxicité.

Un autre inconvénient des solutions proposées dans ces demandes de brevets est de ne pas cibler la zone amont de la matrice de l'ongle qui est un objectif prioritaire des traitements apportés en interne, injectables ou oraux.

Cette zone amont n'est pas non plus ciblée pour les traitements apportés en externe tels que topiques ou vernis.

En résumé, il apparaît que les problèmes thérapeutiques posés par le traitement des maladies de l'ongle et en particulier l'onychomycose, comme le manque de compliance, le manque d'efficacité, la durée et la fréquence de traitement n'ont pas trouvé de solutions satisfaisantes à ce jour.

### RESUME DE L'INVENTION :

La présente invention a donc pour objet une composition pour son utilisation dans le traitement de maladies de l'ongle et/ou pour accélérer la croissance de l'ongle caractérisée en ce qu'elle comprend un ou plusieurs polymères solides biodégradables à libération prolongée, et éventuellement un ou plusieurs principes actifs et en ce qu'elle se présente sous la forme d'un dispositif destiné à être administré par voie sous-cutanée à une distance supérieure à 1cm du bord de l'ongle de préférence dans le doigt à l'exception de la dernière phalange et également hors d'une zone d'articulation, de préférence dans l'avant-dernière phalange éloigné du tendon et de ses zones d'insertion osseuses.

### BREVE DESCRIPTION DES FIGURES :

**Figure** 1: Graphique de libération *In Vitro:*
   Comparaison entre les différentes compositions préparées selon l'Exemple 1.
**Figure** 2: Graphique de libération *In Vitro:*
   Comparaison entre les différentes compositions préparées selon l'Exemple 18.
**Figure** 3: Graphique de libération *In Vitro* des compositions selon l'Exemple 2 et l'Exemple 6.
**Figure** 4: Graphique de libération *In Vitro* de la composition selon l'Exemple 16.
**Figure** 5: Photo du halo d'une plaque d'agar après implantation d'un composé selon l'Exemple 1 (1) sur des semences de souches Candidae Parapsilosis incubées à 35°C
**Figure** 6: Photo du halo d'une plaque d'agar après implantation d'un composé selon l'Exemple 2 sur des semences de souches Candidae Parapsilosis incubées à 35°C
**Figure** 7: photo de halo d'une plaque d'agar après implantation d'un composé selon l'Exemple 19 sur des semences de souches Trichophyton Rubrum incubées à 35°C
**Figure** 8: courbe d'activité du halo d'inhibition d'un implant selon l'Exemple 1 (3) vs sur des semences de souches Trichophyton Rubrum incubées pendant 75 jours à 35°C
**Figure** 9: courbe d'activité du halo d'inhibition d'un implant selon l'Exemple 2 sur des semences de souches Candidae Parapsilosis incubées pendant 168 jours à 35°C
**Figure 10a****:** courbe d'activité du halo d'inhibition d'un implant selon l'Exemple 19 sur des semences de souches Candidae Parapsilosis incubées pendant 115 jours à 27 et 35°C
**Figure 10b** **:** courbe d'activité du halo d'inhibition d'un implant selon l'Exemple 19 sur des semences de souches Trichophyton Rubrum incubées pendant 135 jours à 35°C
**Figure 11** : photo d'un implant de composition décrite à l'exemple 18 (3) après 84 jours d'implantation *In Vivo.*
**Figure 12** : quantité de principe actif libérée en fonction du temps d'un dispositif selon l'Exemple 1 (4)
**Figure 13** : courbe de rémanence en fonction du temps d'un dispositif selon l'Exemple 18 (3)
**Figure 14a****:** Photo d'un implant obtenu suivant l'Exemple 1 (4) - vue de profil
**Figure 14b****:** Photo d'un implant obtenu suivant l'Exemple 1 (4) - vue en coupe
**Figure 15****:** Photo d'un implant obtenu suivant l'Exemple 7 - vue de profil
**Figure 16****:** Photo d'un implant obtenu suivant l'Exemple 8 - vue de profil
**Figure 17****:** Photo d'un implant obtenu suivant l'Exemple 16 - vue de profil
**Figure 18****:** Photo d'un implant obtenu suivant l'Exemple 18 (3) - vue de profil

### DESCRIPTION DETAILLEE DE L'INVENTION :

Par bord de l'ongle autour de la plaque unguéale, on entend plus particulièrement la zone limitée par la cuticule, les replis latéraux et le bord libre sur la partie supérieure du doigt.

Par zone affectée ou zone à traiter, on entend comme zone minimale une extrémité, par exemple l'ongle d'un doigt ou d'un doigt de pied, qui peut s'étendre à plus d'une extrémité, d'un ongle, d'un doigt ou doigt de pied, jusqu'à pouvoir toucher toutes les extrémités, ongles, doigts et doigts de pieds du patient.

L'administration ou les re-administrations seront préférentiellement réalisées sur les faces latéro-supérieures de part et d'autre du tendon extenseur et au-dessus des paquets vasculo-nerveux de ces zones.

La présente invention a donc pour objet une composition pour son utilisation dans le traitement de maladies de l'ongle et/ou pour accélérer la croissance de l'ongle caractérisée en ce qu'elle comprend un ou plusieurs polymères solides biodégradables à libération prolongée, et éventuellement un ou plusieurs principes actifs, et en ce qu'elle se présente sous la forme d'un dispositif destiné à être administré par voie sous-cutanée à une distance supérieure à 1cm du bord de l'ongle de préférence dans le doigt à l'exception de la dernière phalange.

La zone d'administration des compositions selon l'invention se situe de préférence dans l'avant-dernière phalange, de préférence hors d'une zone d'articulation, et de préférence la zone d'administration est éloignée du tendon et de ses zones d'insertion osseuses.

Les polymères ou copolymères solides biocompatibles qui peuvent être utilisés peuvent être choisis parmi les polymères de l'acide lactique (PLA), les polymères de l'acide glycolique, les copolymères acide lactique-acide glycolique (PLGA), les Polycaprolactones (PCL), les copolymères d'acide lactique et de caprolactone, le poly-glycolide-co-lactide-co-caprolactone (PLGC), les Polyanhydrides, le copolymère entre le 1,3-bis (carboxyphénoxypropane) (PCPP) avec acide sébatique (SA), les polyéthylène glycols, les polyorthoesters, les polycarbonates, les polyuréthanes, les polyacétals, les polycyanoacrylates, les polyphosphoesters, les poly(oxyéthylène), les poly(oxypropylène), les polydioxanes ou polydioxanones, les polymères d'acides aminés comme les polyarginines, et les copolymères et mélanges de ces polymères.

Les polymères ou copolymères biocompatibles préférés sont les polymères biodégradables, plus particulièrement les polymères de l'acide lactique (PLA), les polymères de l'acide glycolique, les copolymères acide lactique-acide glycolique (PLGA).

Les compositions selon l'invention peuvent aussi contenir d'autres excipients injectables comme par exemple des acides aminés tels que l'histidine, la lysine ou l'arginine de préférence l'arginine.

En d'autres termes, le premier objet de la présente invention est une composition solide comprenant un ou plusieurs polymères biodégradables à libération prolongée, éventuellement d'autres excipients injectables, et éventuellement un ou plusieurs principes actifs pour son utilisation dans le traitement de maladies de l'ongle et/ou pour accélérer la croissance de l'ongle par administration par voie sous-cutanée à une distance supérieure à 1cm du bord de l'ongle de préférence dans le doigt à l'exception de la dernière phalange et de préférence dans l'avant dernière phalange.

L'objet de la présente invention est donc de faciliter un traitement des maladies de l'ongle et en particulier un traitement des mycoses locales. En particulier, l'objet de l'invention est de proposer un traitement loco-régional, interne, parentéral, c'est à dire injectable de durée prolongée des maladies de l'ongle.

Plus spécifiquement, la présente invention peut consister en de nouvelles utilisations locorégionales de nouvelles formes pharmaceutiques retard ou à relargages prolongés fortement concentrées en principes actifs mais qui correspondent à de faibles doses quotidiennes d'agents antifongiques comparées aux doses délivrées par voie systémique générale ou aux doses disponibles avec les formes topiques d'utilisations locorégionales.

La présente invention permet notamment de soigner les infections par les champignons notamment grâce à l'administration par injection interne sous-cutanée à au moins 1 cm de l'ongle infecté et hors de la dernière phalange qui le porte ou par insertion externe entre la plaque et le lit de l'ongle, d'agents antifongiques selon une posologie inattendue et différente de la posologie habituelle, à des doses très faibles et à des fréquences très réduites, avec une efficacité supérieure.

De plus, de façon surprenante, les inventeurs de la présente demande ont également découvert que l'intervention qui consiste à injecter en sous-cutané, un ou plusieurs implants hors du site de l'ongle porteur de l'onychomycose mais en un endroit proche de ce site, permet un traitement efficace de la maladie de l'ongle et en particulier de l'onychomycose sans qu'il soit nécessaire d'administrer non seulement les hautes doses d'antifongiques classiquement utilisées dans ce type de traitement mais également en l'absence complète de principe actif tels que les antifongiques.

En fonction du type de maladie de l'ongle, un ou plusieurs de tels implants ne comportant pas de principe actif tels que ceux envisagés dans les compositions selon l'invention peuvent être administrés à au moins 1 cm de l'ongle infecté hors de la phalange qui le porte et permettent le traitement de la maladie de l'ongle, en particulier l'onychomycose et des altérations de la morphologie de l'ongle ou onychodystrophie.

Il n'est alors pas nécessaire, mais possible, d'associer ces dispositifs médicaux sous forme d'implants ne contenant pas de principe actif, notamment d'anti fongiques qui constituent aussi des compositions selon l'invention à un ou plusieurs antifongiques, qu'ils soient administrés près de l'ongle malade selon l'invention ou de manière systémique.

On pourra toutefois incorporer une dose totale très faible comparée aux doses nécessaires à ces traitements d'une ou plusieurs de ces molécules afin d'éviter la contamination des dits dispositifs médicaux implantés ou la propagation de la mycose.

Sans que cette tentative d'explication sur le mécanisme par lequel les dispositifs constituant les compositions selon l'invention et ne comportant pas ou seulement très peu de principe actif peuvent guérir une onychomycose puissent constituer une quelconque limitation, on peut supposer que l'implantation d'un tel dispositif peut entraîner un processus inflammatoire et/ou une réaction micromécanique à un corps étranger avec en réponse une mobilisation des facteurs biologiques habituels qui va régénérer la zone d'implantation et les tissus environnants.

Ces processus (tels que la vasodilatation, la présence de macrophages) peuvent aussi être déclenchés par un environnement acide lié aux processus d'élimination du ou des dispositifs implantés biodégradables et lentement résorbables constitués par exemple de polymères biodégradables formés d'acide lactique et/ou acide glycolique, plus communément désignés comme PLA ou PLGA, ce qui pourrait avoir comme résultat de mobiliser des moyens naturels de défenses, d'élimination et/ou de cicatrisation de l'organisme, susceptibles d'entraîner la guérison de l'ongle proche infecté.

Par ailleurs, le dispositif implantable biorésorbable crée grâce à ce micro-environnent localisé proche de la zone à traiter une barrière qui évite aussi la surinfection, contamination ou récidive de colonisation par cet effet micromécanique d'une barrière physique qui empêche la propagation de la maladie en dehors de la zone infectée.

Les inventeurs de la présente demande ont ainsi découvert que de façon surprenante et avantageuse, les maladies de l'ongle et notamment l'onychomycose, peuvent être favorablement traitées par l'implantation sous la peau d'une formulation retard solide, non pas implantée sous l'ongle ou dans les tissus immédiatement environnants, mais à une certaine distance de l'ongle malade dans des sites qui permettent ce type d'injections, plus faciles, plus confortables et non pathogènes.

Par traitement au sens de la présente demande, on inclut la prévention, le contrôle, le traitement ou l'atténuation d'une maladie. On inclut également le traitement de fond, à la naissance ou racine profonde de l'ongle constitué par la matrice, la diminution du risque de récidive, l'arrêt de l'extension de la maladie ainsi que le fait de soulager la douleur.

Le traitement de maladies de l'ongle au sens de la présente invention inclut également l'accélération de la croissance de l'ongle sain en particulier en cas d'onychomycose, ou la restauration de cette croissance dans le cas où la maladie de l'ongle, en particulier l'onychomycose a totalement ou presque totalement interrompu cette croissance.

En effet, un des problèmes majeurs d'efficacité des traitements pour les maladies de l'ongle est lié à la lenteur de la pousse des ongles, encore plus lente dans le cas des ongles « malades ». L'invention a donc pour objet de pouvoir utiliser un excipient ou un principe actif capable de rétablir et/ou accélérer la pousse de l'ongle sain seul ou associé à d'autres excipients ou d'autres principes actifs.

La présente invention a également pour objet l'accélération de la croissance de l'ongle sain en particulier en cas d'absence d'onychomycose.

L'invention selon la présente demande se distingue de l'art antérieur par deux caractéristiques essentielles :
D'une part :
- soit elles ne comportent pas du tout de principe actif en particulier, et dans le cas du traitement de l'onychomycose, elles ne comportent pas d'antifongique,
- soit elles ne comportent qu'une dose totale très faible de principe actif en particulier d'antifongique, dose seulement destinée à éviter la contamination desdites compositions implantées ou la propagation de la mycose, par exemple inférieure à 50%, de préférence inférieure à 20%, de préférence inférieure à 5%, plus préférentiellement inférieure à 2%, encore plus préférentiellement inférieure à 1%.
- soit elles comportent une dose nettement inférieure à la dose habituellement utilisée pour le traitement, en particulier le traitement de l'onychomycose par les antifongiques, par exemple inférieure à 50mg par zone à traiter.

D'autre part, la présente invention permet de soigner les affections par les champignons notamment grâce à l'administration à au moins 1 cm de l'ongle infecté.

Les agents antifongiques lorsqu'ils sont présents sont selon une posologie inattendue et différente de la posologie habituelle, notamment à des doses très faibles et avec une efficacité supérieure.

Dans le cas de la terbinafine par exemple, la posologie prescrite pour les formes orales pour le traitement des maladies de l'ongle est en général de 250 mg par jour, durant 6 à 12 semaines selon la zone infectée, représentant une dose totale de 21 g de principe actif lors d'une durée de traitement de 12 semaines, pour des résultats de seulement 38% de guérison totale.

Avec les mêmes principes actifs, les compositions selon l'invention permettent des traitements prolongés sur 48 semaines et plus, avec des doses totales de terbinafine inférieures à 2g, soit au moins 10 fois inférieures, de préférence inférieures à 200mg soit 100 fois inférieures, plus avantageusement inférieures à 60mg soit 350 fois inférieures, plus avantageusement encore inférieures à 20mg par an soit au moins 1000 fois, et encore plus avantageusement, inférieures à 5mg par an soit au moins 4000 fois plus faibles que celles de ces formes orales actuelles.

Certains actifs comme l'itraconazole peuvent être prescrits pendant 48 semaines à raison de 200mg par jour pour des résultats ne dépassant pas les 15% de patients totalement soignés avec 67 g de principe actif administrés systémiquement.

Dans tous les cas préalablement cités les doses administrées par les compositions selon l'invention peuvent s'ajouter ou se combiner à ces traitements pour améliorer les pourcentages de guérison.

La présente demande a également pour objet le traitement des maladies de l'ongle par des compositions contenant un ou plusieurs principes actifs pour le traitement des maladies des extrémités, et en ce que ces compositions selon l'invention contenant différents principes actifs puissent être administrées de manière alternée dans le temps. A titre d'exemple, il sera possible d'administrer durant une première période de traitement une composition contenant un principe actif tel que la Terbinafine, suivie par une deuxième période de traitement où le patient recevra une composition contenant un deuxième principe actif, par exemple le ciclopirox, qui pourra être suivie par une troisième période de traitement où le patient pourra être réadministré avec une composition contenant à nouveau le premier principe actif, soit une composition contenant un troisième principe actif, par exemple de la cilopiroxolamine, et ceci jusqu'à guérison complète. Il est également possible d'administrer des compositions contenant plusieurs principes actifs à la fois, et de les alterner par périodes de traitement avec des compositions pouvant contenir un seul ou plusieurs principes actifs différents.

De même, il est également possible d'administrer un ou plusieurs implants en même temps, chacun contenant un principe actif (ou plus) différent, et d'ensuite alterner avec un ou plusieurs implants en même temps, chacun contenant un principe actif (ou plus), qui peuvent être, ou non, différents des premiers principes actifs implantés.

D'autre part, les compositions selon l'invention sont administrées, non pas de façon systémique sous l'ongle ou dans les tissus immédiatement environnants, mais à une distance supérieure à 1cm du bord de l'ongle et de préférence en dehors de la dernière phalange ou d'une zone d'articulation, de préférence dans l'avant-dernière phalange.

Les doses délivrées d'excipients et de principes actifs sont proportionnelles à la taille totale des formulations solides injectées.

Ces nouvelles zones d'administration permettent de délivrer des doses quotidiennes plus élevées que celles compatibles avec l'art antérieur à partir de formulations de plus grands volumes, plus faciles, plus confortables et moins douloureuses que celles réalisées trop proche ou dans les zones traumatiques et particulièrement sensibles de l'ongle.

On pourra par exemple injecter une ou plusieurs formulations solides de diamètre supérieur à 0,60 mm et de longueur supérieure à 1 cm. De telles tailles de formulations solides nécessitent, pour être administrées sous l'ongle ou dans les tissus immédiatement environnants, une aiguille de diamètre supérieur à 0,90 mm. On comprend aisément que l'aiguille serait incompatible avec des injections dans ces zones trop petites, et serait extrêmement douloureuse pour le patient.

Il est donc facile de constater que les formes et volumes envisagés dans les compositions selon l'invention ne seraient en aucun cas compatibles avec les zones d'administration des approches de traitements proposées dans l'art antérieur.

La présente invention a donc ainsi pour objet des compositions pour leurs utilisations dans le traitement de maladies et/ou altérations de la morphologie de l'ongle et/ou pour accélérer la croissance de l'ongle, et en ce que ces compositions comprennent un ou plusieurs principes actifs. En particulier, la présente invention concerne une composition comprenant un ou plusieurs principes actifs choisis parmi :
- Les antis infectieux choisis parmi :
   les agents antifongiques,
   les antibiotiques
   et les antiparasitaires
- Les anti-inflammatoires choisis parmi
   les anti-inflammatoires stéroïdiens
   les anti-inflammatoires non stéroïdiens et
   les inhibiteurs de Janus Kinase (JAK),
- Les ciclosporines,
- la vitamine D ou les dérivés de la vitamine D,
- Les immunosuppresseurs,
- Les acides aminés, de préférence l'Arginine,
- Les principes actifs accélérant la croissance de l'ongle choisis de préférence parmi :
   les prostaglandines.
   le Chitosan,
   l'Acide valproïque
   l'Echisetum arvense (la prêle), l'Acide hyaluronique, la Biotine, l'Hormone de croissance (GH), le Minoxidil, la Finasteride.
- Les principes actifs favorisant la circulation sanguine choisis parmi
   les vasodilatateurs et
   les anti agrégants plaquettaires,
- Les anesthésiques locaux,
   pour son utilisation dans le traitement de maladies de l'ongle par administration par voie sous-cutanée à une distance supérieure à 1cm du bord de l'ongle de préférence dans le doigt à l'exception de la dernière phalange.

La présente invention a donc pour objet une composition telle que décrite ci-dessus et comprenant de préférence un ou plusieurs principes actifs choisis parmi :
- Les agents antifongiques, choisis parmi la Nystatine, l'Amphotéricine B, l'Abafungine, le Benzalkonium chloride, la Caspofungine, le Cetrimide, le Clioquinol, le Sulphate de cuivre, l'Haloprogine, les Echinocandines, la Flucytosine, le Mycobactovir, la Novexatine, la Natamycine, le Cetylpyridium chlorure, la Benzylamine butenafine, les Benzoxaboroles (dérivés cycliques des acides boroniques), l'Albaconazole, l'Arasertaconazole, le Bifonazole, le Butoconazole, le Clotrimazole, l'Eberconazole, l'Éconazole, l'Eficonazole, le Fenticonazole, le Fluconazole, le Fosravuconazole, l'Imidazole, l'Isoconazole, l'Irtemazole, l'Isavuconazole, le Ketoconazole, le Liarozole, le Lianozole, le Luliconazole, le Miconazole, l'Oxadiazole, l'Oxiconazole, le Posaconazole, le Pramiconazole, le Ravuconazole, le Sertaconazole, le Sulconazole, le Terconazole, le Thiazole, le Thiabendazole, le Thiadiazole, le Thiamazole, le Tioconazole, le Voriconazole, l'Arginine, l'Amiodarone, la Naftifine, le Tavaborole, la Butenafine, la Flucytosine, la Griséofulvine, la Caspofungine, la Micafungine, l'Oxide Nitrique, l'Oxychlorosène de sodium, la Povidoneiodine, le Thiocarbonate tolnafate, la Sulbentine, le Zync Pyrithione, et plus particulièrement parmi la Terbinafine, le Ciclopirox, la Ciclopiroxolamine, l'Itraconazole, le Miconazole, et l'Amorolfine.
   Préférentiellement, les antifongiques sont choisis parmi la terbinafine éventuellement sous forme de chlorhydrate, le ciclopirox, le ciclopirox olamine, l'amorolfine et le Miconazole.
   Notamment pour le traitement des infections par pseudomonas, les compositions selon l'invention peuvent comporter un antibiotique, de préférence les Fluoroquinoles,
   Les compositions de l'invention peuvent aussi comporter des antiparasitaires qui peuvent être choisis parmi Emamectine, Selamectine, Imidacloprid, moxidectin, Milbemycin oxime, Lufenuron, Fumagillin, les Antiamoebics : Rifampicin, Amphotericin B ; les Antihelmintics : Fenbendazole, Triclabendazole, Flubendazole, Abamectin, Suramin, Levamisole, Niclosamide, Nitazoxanide, Oxyclozanide, les dérivés d'Aminoacétonitrile, Spiroindoles, Pelletierine sulphate et Artemisinin
- Pour le traitement des mycoses et en particulier de l'onychomycose, il peut être avantageux d'administrer, en utilisant les compositions de l'invention, un anti-inflammatoire en même temps qu'un ou plusieurs des produits antifongiques mentionnés ci-dessus.
   Les anti-inflammatoires stéroïdiens qui peuvent être administrés en même temps que les antifongiques peuvent être choisis parmi les suivants : Hydrocortisone, Methylprednisolone, Prednisolone, Prednisone, Amcinonide, Budesonide, Desonide, Fluocinolone acétonide, Fluocinonide, Halcinonide, Beclometasone, Betamethasone, Fluocortolone, Halometasone, Mometasone, Alclometasone dipropionate, Betamethasone dipropionate and valerate, Clobetasol propionate and butyrate, Fluprednidene acetate, Mometasone furoate, Ciclesonide, Cortisone acetate, Hydrocortisone sous forme d'aceponate, acetate, buteprate, butyrate et valerate, Prednicarbate, et Tixocortol pivalate, de préférence, la Dexaméthasone ou la Triamcinolone.
   Les anti-inflammatoires non stéroïdiens qui peuvent être administrés en même temps que les antifongiques peuvent être choisis parmi les suivants : l'Acide Acétylsalicylique et l'Acétylsalicylate de lysine, le Salicylate de méthyle, le Diflunisal, les Arylalkanoïques, les Profènes, les dérivés Indoliques, les Oxicams, les CINODs, les Sulfonanilides, le groupe des Coxibs, le Phénylbutazone, l'Acide niflumique et les Acides Phénamiques.
   Un autre type d'anti-inflammatoires pouvant être administré sont les inhibiteurs de Janus Kinase (JAK),
- Pour le traitement des maladies inflammatoires et en particulier le psoriasis, accompagné ou non de phénomènes inflammatoires, on peut également utiliser un des anti-inflammatoires cités précédemment.
- En plus d'un ou plusieurs anti-inflammatoires, le traitement des maladies de l'ongle et en particulier le traitement des maladies inflammatoires, comme le psoriasis peut être effectué à l'aide des compositions de l'invention comprenant une ou plusieurs ciclosporines, de préférence la ciclosporine A, la vitamine D ou des dérivés de la vitamine D, de préférence le calcipotriol ou le paricalcitol,
- Les compositions de l'invention peuvent également comporter des immunosuppresseurs, de préférence le methotrexate, des rétinoïdes, de préférence le Tazarotene, ou encore des composés tels que l'Anthralin (ou dithranol) et l'Urée.
- Les compositions selon l'invention peuvent également comporter un ou plusieurs acides aminés, de préférence l'Arginine.
- Les compositions de l'invention peuvent également comporter un ou plusieurs principes actifs accélérant la croissance de l'ongle choisis de préférence parmi les prostaglandines. Les prostaglandines pouvant être utilisées peuvent être choisies de préférence parmi les dérivés de la prostaglandine F2α ou les pro-drugs de PGF2alpha et notamment la latanoprost, la travoprost ou la bimatoprost, plus préférentiellement encore le latanoprost ou le bimatoprost.
   Pour favoriser la croissance des ongles on peut également utiliser le Chitosan l'Acide valproïque l'Echisetum arvense (la prêle), l'Acide hyaluronique, la Biotine, l'Hormone de croissance (GH), le Minoxidil et/ou le Finasteride.
- Les compositions de l'invention peuvent également comporter un ou plusieurs principes actifs favorisant la circulation sanguine tels que les vasodilatateurs et les anti agrégants plaquettaires. Les vasodilatateurs, peuvent être choisis parmi Amlodipine (besylate, mesylate or maleate), Nifedipine, Diltiazem, Vérapamil, Ginkgo biloba, Iloprost, Endotheline, Nacitentan, Bosentan, Acide nicotinique (Niacine), Arginine, Oxyde nitrique, Nitroglycérine, Dinitrate d'isosorbide, Prostacycline (PGI2) et d'autres prostaglandines, Histamine, Bradykinine.
- Notamment pour le traitement des complications du pied diabétique, maladie de Buerger, maladie de Raynaud, artérite des membres inférieurs, on peut utiliser un anti agrégant plaquettaire tel que le Clopidogrel,
- Les compositions de l'invention peuvent aussi comporter des anesthésiques locaux qui peuvent être choisis parmi la Lidocaine, Articaine, Bupivacaine, Lebobupivacaine, Mepivacaine, Procaine et Ropivacaine,
- On utilise de préférence des compositions contenant de l'acide valproïque qui est une substance généralement connue et utilisée dans le traitement des maladies tenant à à un déficit en kératine telle que l'alopécie androgénique,
- La présente invention a particulièrement pour objet une composition telle que décrite ci-dessus pour son utilisation dans le traitement de maladies de l'ongle, caractérisée en ce que la maladie de l'ongle est l'onychomycose et en ce que la composition comprend un ou plusieurs principes actifs mentionné ci-dessus choisis parmi :
   - les agents antifongiques choisis de préférence parmi la terbinafine éventuellement sous forme de chlorhydrate, le ciclopirox, la ciclopiroxolamine , l'amorolfine et le Miconazole,
   - les anti-inflammatoires stéroidiens ou non stéroïdiens, choisis de préférence parmi la dexamethasone et la triamcinolone
   - les ciclosporines,
   - les acides aminés, de préférence l'Arginine,
   - les principes actifs accélérant la croissance de l'ongle, notamment le chitosan
   - les vasodilatateurs
   - les anesthésiques locaux
   - les antibiotiques
   - les antiparasitaires, notamment l'emamectine et la selamectine

Les compositions selon l'invention que l'on utilise pour traiter les maladies inflammatoires indiquées ci-dessus et en particulier le psoriasis peuvent avantageusement contenir une ou plusieurs ciclosporines telles que les ciclosporines A ou G, de préférence, la ciclosporine A.

La présente invention a donc également particulièrement pour objet une composition telle que décrite ci-dessus pour son utilisation dans le traitement de maladies de l'ongle, caractérisée en ce que la maladie de l'ongle est le psoriasis accompagné ou non d'un phénomène inflammatoire et en ce que la composition comprend un ou plusieurs principes actifs choisis de préférence parmi :
- Les anti-inflammatoires stéroïdiens parmi : Hydrocortisone, Methylprednisolone, Prednisolone, Prednisone, Amcinonide, Budesonide, Desonide, Fluocinolone acétonide, Fluocinonide, Halcinonide, Beclometasone, Betamethasone, Fluocortolone, Halometasone, Mometasone, Alclometasone dipropionate, Betamethasone dipropionate and valerate, Clobetasol propionate and butyrate, Fluprednidene acetate, Mometasone furoate, Ciclesonide, Cortisone acetate, Hydrocortisone sous forme d'aceponate, acetate, buteprate, butyrate et valerate, Prednicarbate, et Tixocortol pivalate, de préférence, la Dexaméthasone ou la Triamcinolone.
- Les anti-inflammatoires non stéroïdiens parmi : l'Acide Acétylsalicylique et l'Acétylsalicylate de lysine, le Salicylate de méthyle, le Diflunisal, les Arylalkanoïques, les Profènes, les dérivés Indoliques, les Oxicams, les CINODs, les Sulfonanilides, le groupe des Coxibs, le Phénylbutazone, l'Acide niflumique et les Acides Phénamiques.
- Les inhibiteurs de Janus Kinase (JAK),
- une ou plusieurs ciclosporines, de préférence la ciclosporine A,
- La vitamine D ou des dérivés de la vitamine D, de préférence le calcipotriol ou le paricalcitol,
- des vaso-dilatateurs,
- des immunosuppresseurs, de préférence le methotrexate,
- des rétinoïdes, de préférence le Tazarotene,
- des composés tels que l'Anthralin (ou dithranol) et l'Urée.
- les principes actifs accélérant la croissance de l'ongle choisis parmi les prostaglandines de préférence le latanoprost ou le bimatoprost ;
- le chitosan
- l'acide valproïque.
- Les vasodilatateurs
- Les anesthésiques locaux
- L'Arginine

Pour les traitements des complications du pied diabétique, maladie de Buerger, maladie de Raynaud, artérite des membres inférieurs, on peut ajouter un anti agrégant plaquettaire tel que le Clopidogrel à la composition selon l'invention, ainsi que des vasodilatateurs, choisis parmi Amlodipine (besylate, mesylate or maleate), Arginine, Nifedipine, Diltiazem, Vérapamil, Ginkgo biloba, Iloprost, Endotheline, Nacitentan, Bosentan, Acide nicotinique (Niacine), Oxyde nitrique, Arginine, Nitroglycérine, Dinitrate d'isosorbide, Prostacycline (PGI2) et d'autres prostaglandines, ou encore par exemple de l'Histamine et de la Bradykinine.

Lorsque seule la croissance de l'ongle est recherchée, les compositions selon l'invention peuvent comporter un ou plusieurs principes actifs choisis parmi les ciclosporines, les prostaglandines, de préférence, les ciclosporines et les prostaglandines mentionnées ci-dessus, le chitosan et/ou l'acide valproïque.

La présente invention a également particulièrement pour objet une composition telle que décrite ci-dessus pour son utilisation pour accélérer la croissance de l'ongle caractérisée en ce qu'elle comprend un ou plusieurs principes actifs choisis parmi :
- les ciclosporines de préférence la ciclosporine A,
- les prostaglandines choisies de préférence parmi le latanoprost ou le bimatoprost,
- l'Echisetum arvense (la prêle), le Chitosan, l'Acide hyaluronique, la Biotine, l'Acide valproïque, l'Hormone de croissance (GH), le Minoxidil et/ou le Finasteride
- les acides aminés, de préférence l'Arginine,
- les vasodilatateurs, choisis par exemple parmi le gingko biloba, l'oxyde nitrique et l'arginine
- l'acide valproïque.

En résumé, pour le traitement des mycoses et en particulier de l'onychomycose, il peut être avantageux d'administrer, en utilisant les compositions de l'invention, un anti-inflammatoire, de préférence un anti-inflammatoire stéroïdien en même temps qu'un ou plusieurs des produits antifongiques mentionnés ci-dessus.

Une composition selon l'invention peut comporter de préférence :
Un ou plusieurs produits antifongiques choisis parmi les produits suivants :
Ciclopiroxolamine, Amorolofine, Imidazoles, Tolnaftate, Bifonazole, Butenafine, Tioconazole, terbinafine, Itraconazole, Fluconazole, Griseofulvine, Ketoconazole, Cyclopiroxamine, Miconazole et plus particulièrement parmi la Terbinafine, la Cyclopiroxolamine, l'itraconazole et l'amorolfine.

Un ou plusieurs anti-inflammatoires qui peuvent être administrés en même temps que les antifongiques sont choisis de préférence parmi :
- Les anti-inflammatoires stéroïdiens parmi : Hydrocortisone, Methylprednisolone, Prednisolone, Prednisone, Amcinonide, Budesonide, Desonide, Fluocinolone acétonide, Fluocinonide, Halcinonide, Beclometasone, Betamethasone, Fluocortolone, Halometasone, Mometasone, Alclometasone dipropionate, Betamethasone dipropionate and valerate, Clobetasol propionate and butyrate, Fluprednidene acetate, Mometasone furoate, Ciclesonide, Cortisone acetate, Hydrocortisone sous forme d'aceponate, acetate, buteprate, butyrate et valerate, Prednicarbate, et Tixocortol pivalate, de préférence, la Dexaméthasone ou la Triamcinolone.
- Les anti-inflammatoires non stéroïdiens parmi : l'Acide Acétylsalicylique et l'Acétylsalicylate de lysine, le Salicylate de méthyle, le Diflunisal, les Arylalkanoïques, les Profènes, les dérivés Indoliques, les Oxicams, les CINODs, les Sulfonanilides, le groupe des Coxibs, le Phénylbutazone, l'Acide niflumique et les Acides Phénamiques.
- Les inhibiteurs de Janus Kinase (JAK),

Un ou plusieurs vasodilatateurs, qui peuvent être choisis parmi Amlodipine (besylate, mesylate or maleate), Nifedipine, Arginine, Diltiazem, Vérapamil, Ginkgo biloba, Iloprost, Endotheline, Nacitentan, Bosentan, Acide nicotinique (Niacine), Oxyde nitrique, Nitroglycérine, Dinitrate d'isosorbide, Prostacycline (PGI2) et d'autres prostaglandines, Histamine, Bradykinine,

Un anesthésique local tel que la lidocaïne.

Les compositions selon l'invention peuvent revêtir n'importe quelle forme ou taille qui soit compatible avec les zones spécifiques nouvelles d'administration et avec le déclanchement d'une réaction locale de l'organisme qui soit capable de traiter la maladie de l'ongle en l'absence de principe actif tel qu'un antifongique ou la faculté de délivrer la quantité suffisante de principe actif pour être thérapeutiquement efficace dans le traitement de la maladie de l'ongle et/ou l'accélération ou le rétablissement de la croissance ou la repousse de l'ongle.

Les compositions peuvent se présenter sous la forme d'un implant présentant éventuellement une extrémité aiguisée ou d'un tube cylindrique, d'une barre ou d'un fil. Elles peuvent également être sous forme de particules, de disques ou de feuilles.

Les compositions de l'invention peuvent par exemple être administrées à l'aide d'une aiguille ou d'un dispositif qui évite totalement l'usage d'une aiguille et avec lequel, les compositions sont directement injectées sous la peau du patient. Des dispositifs de ce type, les compositions qui peuvent être ainsi administrées et leur procédé de préparation sont par exemple décrits dans la demande internationale WO 96/08289.

Les compositions selon l'invention peuvent par exemple être comparables à un fil de suture mono- ou multi-filaments de PLA et PLGA, de préférence d'une longueur d'au moins 10 mm et jusqu'à 25mm. Ces fils flexibles peuvent être mis en place à l'aide d'une aiguille et ont l'avantage de pouvoir épouser la forme du doigt.

Selon les différentes formes que peuvent prendre les compositions de l'invention (implants, fils) on peut implanter par exemple deux ou plusieurs implants ou fils de chaque côté du doigt, éventuellement de façon symétrique et préférentiellement sur les parties dorsales externes de l'avant dernière phalange.

Sous forme de fils, des compositions de l'invention pourront avoir une plus grande longueur, supérieure à 25mm, et par exemple de 10 cm, de façon à pouvoir implanter en les coupant aux longueurs souhaitées, à partir d'une même aiguille, par exemple de chaque côté du doigt à traiter ou l'un à côté de l'autre dans un même doigt.

Les compositions peuvent avantageusement comporter une extrémité pointue ayant la forme d'un cure-dent et avoir une dureté suffisante pour leur permettre de pénétrer à travers la peau dans les couches sous-cutanées.

La présente invention a également particulièrement pour objet une composition telle que décrite ci-dessus pour son utilisation dans le traitement de maladies de l'ongle et/ou pour accélérer la croissance de l'ongle caractérisée en ce qu'elle se présente sous la forme d'un implant, d'un tube cylindrique ou d'un fil, présentant éventuellement une extrémité aiguisée et en ce que la composition est administrée dans le doigt à l'exception de la dernière phalange.

Selon un mode préféré d'exécution de l'invention, les compositions selon l'invention se présentent sous la forme d'implants comprenant un ou plusieurs principes actifs et un ou plusieurs polymères, biocompatibles, biodégradables ou biorésorbables.

Par biocompatibles, on entend un polymère qui ne provoque pas d'irritation des tissus environnants.

Par biodégradables on entend des polymères qui se dégradent avec le temps sous l'action des enzymes ou des hydrolyses.

Bioabsorbables ou bioresorbable signifie que le ou les polymères constitutifs des compositions sont métabolisés et sont absorbés par les tissus environnants.

Dans un mode de réalisation préféré de l'invention, des copolymères d'acide lactique et glycolique (PLGA) sont utilisés. Le pourcentage de chaque monomère peut varier de 0 à 100%. Il peut par exemple être d'environ 50/50 mais peut être aussi d'environ 15-85%, 25-75%, 35-65%, 0-100%.

Les polymères et copolymères peuvent être coiffés (capped) à leurs extrémités par une fonction acide ou une fonction ester.

Les polymères solides à libération prolongée préférés sont les polymères de l'acide L-lactique, de l'acide D-lactique, de l'acide DL-lactique, du L-lactide, du D-lactide, du DL-lactide, du glycolide, de l'acide glycolique, de la caprolactone, de la poly(dioxanone) et tous les copolymères correspondants.

Le polymère de PLA préféré est un poly (D,L-lactide) de viscosité comprise entre 0,16 et 0,75dL/g.

Le copolymère de PLGA préféré est un 85:15 de viscosité comprise entre 0,15 et 1,7dL/g, de préférence de l'ordre de 0,25 et 1,3 dL/g.

La charge en principe actif peut varier entre 0 et 98%, en particulier être inférieure à 85%, de préférence inférieure à 75%, elle est préférentiellement comprise entre 20 et 65%. Elle peut en particulier être de 20, 35, 40, 50, 60 ou 65%.

Comme indiqué ci-dessus, les compositions selon l'invention peuvent ne pas comporter du tout de principe actif (composition « placebo »). Elles peuvent également comporter une très faible dose de principe actif par exemple un antifongique pour éviter la contamination desdites compositions implantées ou la propagation de la mycose. Dans ce dernier cas, la concentration peut-être de l'ordre de 0.01%

La quantité de principe actif, de préférence la Terbinafine chlorhydrate, la ciclopiroxolamine ou le Ciclopirox, par implant qui est la forme préférée des compositions de l'invention peut être comprise entre 0 et 6,5 mg, plus particulièrement de 0,01 à 2,5 mg de produit. Cette quantité de principe actif peut être par exemple de 0.01, 0.02, 0.04, 0.16, 0.20, 0.30, 0.5, 0.78, 1.00, 1.10, 1.20, 1.30, 1.40, 1.50, 1.60, 1.70, 1.80, 1.90, 2.00, 2.10, 2.20, 2.30, 2.40, 2.50, 2.60, 2.70, 2.80, 2.90, 3.00, 3.10, 3.20, 3.30, 3.40, 3.50, 3.60, 3.70, 3.80, 3.90, 4.00, 4.10, 4.20, 4.30, 4.40, 4.50, 4.60, 4.70, 4.80, 4.90, 5.00, 5.10, 5.20, 5.30, 5.40, 5.50, 5.60, 5.70, 5.80, 5.90, 6.00, 6.10, 6.20, 6.30, 6.40 ou 6.50 mg.

La présente invention a particulièrement pour objet une composition telle que décrite ci-dessus caractérisée en ce que le polymère solide à libération prolongée est un polyester de préférence un copolymère acide lactique-acide glycolique (PLGA) ou un polymère de l'acide lactique (PLA) et en ce que la charge en principe actif est inférieure ou égale à 85% et de préférence comprise entre 20 et 65% en poids.

La présente invention a particulièrement pour objet une composition telle que décrite ci-dessus caractérisée en ce qu'elle comprend entre 0,04 et 6.50 mg de chlorhydrate de terbinafine ou de ciclopirox et du PLGA.

La présente invention a particulièrement pour objet une composition telle que décrite ci-dessus caractérisée en ce qu'elle comprend entre 0.04 et 6.50 mg de ciclopirox ou ciclopiroxolamine et du PLGA.

Les compositions selon l'invention peuvent avoir un volume total par implant ou injection d'au plus environ 30 mm³, leur volume peut être compris entre environ 5 et environ 30 mm³, préférentiellement entre environ 10 et environ 30 mm3. Elles peuvent cependant avoir un volume total de plus de 30 mm³.

Le diamètre de l'implant peut être compris entre 0,1 et 1 mm, de préférence entre 0,1 et 0,9, de préférence encore entre 0,3 et 0,8 mm et notamment le diamètre peut être de 0,1, 0,25, 0,3, 0,4, 0,45, 0,50, 0,55, 0,60, 0,65, 0,85 ou 0,90 mm.

La longueur de l'implant par injection peut être au maximum de 25 mm, elle est de préférence comprise entre 4 et 15 mm, elle peut être par exemple de 4, 6, 8, 9 ou 10 mm. La longueur totale peut-être supérieure à 25 mm et l'implant coupé aux mesures nécessaires à chaque implantation.

La présente invention a donc pour objet une composition pour son utilisation dans le traitement des mycoses de l'ongle aussi appelée onychomycose caractérisée en ce qu'elle comprend un ou plusieurs polymères solides biodégradables à libération prolongée, et éventuellement un ou plusieurs antifongiques et en ce qu'elle se présente sous la forme d'un implant ayant une longueur maximale injectée de 25 mm de préférence entre 4 et 15 mm et un diamètre compris entre 0,1 et 1 mm de préférence entre 0,3 et 0,8 mm, implant destiné à être administré par voie sous-cutanée à une distance supérieure à 1cm du bord de l'ongle dans le doigt à l'exception de la dernière phalange.

En d'autres termes, la présente invention a donc pour objet une composition sous forme d'implant ayant une longueur maximale injectée de 25 mm de préférence entre 4 et 15 mm et un diamètre compris entre 0,1 et 1 mm de préférence entre 0,3 et 0,8 mm comprenant un ou plusieurs polymères solides biodégradables à libération prolongée et un ou plusieurs antifongiques pour son utilisation dans le traitement de l'onychomycose par administration par voie sous-cutanée à une distance supérieure à 1cm du bord de l'ongle de préférence dans le doigt à l'exception de la dernière phalange

L'invention a donc aussi pour objet, une composition décrite ci-dessus pour son utilisation dans le traitement de maladies de l'ongle et/ou pour accélérer la croissance de l'ongle caractérisée en ce qu'elle se présente sous la forme d'un implant, d'un tube cylindrique ou d'un fil, présentant éventuellement une extrémité aiguisée, et en ce que la composition est administrée dans le doigt à l'exception de la dernière phalange, dans la première ou la deuxième phalange des doigts, de préférence la deuxième phalange et dans la première phalange du pouce ou du gros orteil et en ce qu'une ou plusieurs compositions, de préférence de une à huit compositions, plus préférentiellement de une à six compositions sont administrées simultanément dans le même doigt.

L'invention a spécialement pour objet la composition décrite ci-dessus caractérisée en ce que le traitement est continu, de préférence pendant une durée minimale de 48 semaines lorsque la totalité de l'ongle est affecté, une durée minimale de 24 semaine lorsque moins de la moitié de l'ongle est affecté, ou encore une durée minimale de 12 semaines lorsque moins du quart de l'ongle est affecté ou encore une durée de 6 semaines lorsque moins de 10% de l'ongle est affecté.

De manière à éviter tout doute, il est précisé que les compositions selon l'invention peuvent, lorsqu'elles sont utilisées dans les applications revendiquées, contenir un ou plusieurs principes actifs choisis parmi n'importe lequel des principes actifs mentionnés ci-dessus.

Il est possible de mélanger différents principes actifs dans un même implant ou un même fil. Il est aussi possible de réaliser des implants ou fils avec chaque principe actif et de combiner l'administration de deux ou plus de ces implants et fils selon les besoins spécifiques de chaque patient, par exemple en fonction des types de champignons impliqués dans leurs mycoses, ou pour traiter l'inflammation liée à la mycose en même temps que le champignon causant la mycose.

L'administration des compositions selon l'invention est effectuée par voie sous-cutanée. Comme indiqué ci-dessus, on peut utiliser un dispositif approprié pour réaliser cette administration et un exemple d'un tel dispositif est décrit dans la demande internationale WO 96/08289.

L'invention a donc pour objet une composition telle que décrite ci-dessus pour son administration par voie sous-cutanée dans la première ou la deuxième phalange des doigts, de préférence l'avant-dernière phalange soit la deuxième phalange et dans la première phalange du pouce ou du gros orteil.

Les compositions selon la présente invention sont donc administrées ou injectées en s'éloignant de l'extrémité du doigt et à une distance supérieure à 1cm de la cuticule, et en particulier elles sont administrées par voie sous-cutanée hors des phalanges distales, dans les phalanges intermédiaires ou proximales ou pour les pieds, les phalanges proximales ou première ou la deuxième phalange des doigts, de préférence la deuxième phalange et dans la première phalange du pouce ou du gros orteil.

De préférence ces compositions sont administrées sur les parties dorsales latérales des doigts ou de chaque côté des faces dorsales et non palmaires de l'avant dernière phalange, au milieu de cette phalange à égale distance des deux articulations qui la délimitent.

Le caractère nouveau et inventif de la présente invention réside notamment dans le fait que l'administration ou l'injection des compositions selon l'invention n'est pas effectuée sous l'ongle ou à proximité immédiate de l'ongle comme cela est pratiqué avec les injections connues pour ces types de traitements et comme cela a été proposé dans plusieurs demandes de l'art antérieur, notamment dans les documents suivants : brevet USP 8.747820, les demandes internationales WO2011/087867 ou WO2006/063350 mais à une distance supérieure à 1cm du bord de l'ongle hors de la phalange qui le porte.

Comme indiqué ci-dessus, l'administration ou l'injection dans les tissus vivants, en dehors de la zone malade de l'ongle ou de son environnement immédiat évite de perturber une zone traumatisée par la mycose, perturbation qui retarde la guérison. De plus, cette zone non affectée est plus grande et il est plus facile et beaucoup moins douloureux d'y faire une injection que dans la zone de l'ongle et cet espace permet d'administrer plusieurs implants si nécessaire.

D'un autre côté, le faible volume disponible en injection sous l'ongle ou dans la proximité immédiate de l'ongle ne permet pas en général de délivrer une dose de principe actif suffisante pour le traitement envisagé : cela ne permet pas de cibler à la base de la croissance ou du renouvellement de l'ongle que constitue la matrice.

L'invention a donc pour objet une composition telle que décrite ci-dessus et caractérisée en ce qu'une ou plusieurs compositions, de préférence de une à six compositions, plus préférentiellement de 1 à 4 compositions sont administrées simultanément dans le même doigt.

Cette zone en amont de l'ongle permet de diriger les traitements injectés préférentiellement ou en priorité vers la matrice à la source profonde de l'ongle, dans une zone inaccessible aussi aux formes topiques externes. Cela permet d'envisager le traitement de fond des mycoses par exemple en cas d'infection de l'ensemble de l'ongle, sans présenter les inconvénients des formes orales et donc sur des périodes de temps beaucoup plus longues jusqu'à pouvoir couvrir les besoins après plus d'un an pour éviter une récidive ou réinfection.

Pour pratiquer cette injection ou ce dépôt en évitant que ce traitement soit douloureux pour le patient, on pourra préalablement pratiquer une anesthésie locale, topique ou injectable, de la zone d'implantation sous-cutanée.

Le traitement spécifique d'administration pourra être réalisé immédiatement et rapidement par un dermatologue ou podologue spécialisé. Il évite au patient tout traitement local par exemple avec des crèmes, traitement souvent rendu difficile avec l'âge, long et compliqué à appliquer au malade. Il assure le suivi du traitement qui ne dépend plus de l'observance par le malade.

Le dépôt pourra être sous cutané ou intra dermique.

Comparée aux traitements LASER comportant parfois un perçage de l'ongle, l'administration des compositions selon l'invention permet d'obtenir un meilleur résultat avec beaucoup moins de contraintes et pour un coût très inférieur.

Cependant, de nouveaux procédés de traitement des maladies de l'ongle et en particulier de l'onychomycose ont été développés plus récemment. Ces traitements consistent à provoquer la rupture des spores et la destruction du champignon par effet thermique photobiologique de la tablette unguéale par LASER. Le traitement est généralement à réitérer au moins 3 fois en laissant 4 à 6 semaines entre chaque traitement.

Pendant le traitement au LASER, un faisceau LASER à une longueur d'onde contrôlée est dirigé vers le lit de l'ongle, générant de la chaleur sous l'ongle par effet thermique à une longueur d'onde déterminée pour détruire la colonie fongique.

Des résultats obtenus avec cette technique ont été rapportés par exemple dans la publication suivante : Novel LASER Therapy in treatment of Onychomycosis, Jasmina Kozarev et al. Journal of the Laser and Health Academy, Vol. 2010, N°1; p. 1

Plusieurs appareils générateurs de rayons LASER commerciaux peuvent être utilisés, les appareils PinPointe™, FootLASER™ ou ND:YAG ou en particulier l'appareil S30 PODYLAS ™ . Les longueurs d'ondes couramment utilisées sont de 870, 930 ou particulièrement 1064 nM

Cependant, ces traitements LASER ne permettent pas toujours d'atteindre les zones infectées profondes et leur efficacité peut donc être limitée et leur coût, onéreux, peut être une limitation pour leur utilisation.

Les compositions selon l'invention pourront donc utilement se combiner au traitement LASER pour atteindre les zones infectées profondes inaccessibles au LASER et en améliorer l'efficacité.

Il est clair que si l'on peut envisager pour ces traitements des injections répétées, l'approche la mieux adaptée est celle d'une injection dépôt unique ou d'un minimum d'injection qui vont ensuite libérer le ou les principes actifs, notamment antifongiques, localement sur une période suffisante, compatible avec le traitement complet, de préférence de plusieurs mois à 1 an.

De façon avantageuse, il est donc possible grâce à l'invention d'administrer une fois par an, par semestre, par trimestre, par mois ou par semaine selon la gravité de l'infection, une dose de principe actif de 0 à 50 mg par zone affectée, ou de différents principes actifs.

Il est possible de multiplier les traitements et administrations pour un même traitement et ainsi couvrir au mieux les nécessités des patients en fonction du type de champignons et autres maladies de l'ongle concomitantes sans administrer des hautes doses ou les doses habituelles thérapeutiques utilisées par voie orale ou topique.

De préférence, l'administration du ou des principes actifs à l'aide des compositions de l'invention est réalisée pendant au moins 48 semaines. De préférence cette administration est réalisée pendant plus de 48 semaines.

De préférence cette administration remplace les traitements habituels à hautes doses, délivrés par la voie orale au début des cas de mycoses sévères et peut être poursuivi tout au long du traitement jusqu'à guérison même après un an ou lorsque les autres traitements par exemple topiques sont interrompus, pour éviter une réinfection de la matrice et les risques de récidives.

L'invention a donc pour objet une composition telle que décrite ci-dessus pour son utilisation dans le traitement de maladies de l'ongle caractérisée en ce qu'une ou plusieurs compositions sont administrées simultanément dans le même doigt et en ce que le traitement est continu, de préférence pendant une durée minimale de 48 semaines. De préférence les administrations ont lieu tous les mois, tous les trois mois ou tous les six mois, et le traitement est délivré sur au moins douze mois, voire sur dix-huit mois.

Un autre objet de l'invention est de traiter certaines maladies de l'ongle dans lesquelles la totalité de l'ongle n'est pas touchée, mais seulement une partie est infectée ou malade. Dans ces cas spécifiques, la durée de traitement sera inférieure à 48 semaines ; par exemple la durée de traitement minimale pourra être de 24 semaines si une superficie inférieure à la moitié de l'ongle est affectée par la maladie, ou encore la durée de traitement minimale pourra être de 12 semaines si une superficie inférieure au quart de l'ongle est affectée par la maladie.

L'invention a donc pour objet une composition telle que décrite ci-dessus pour son utilisation dans le traitement de maladies de l'ongle caractérisée en ce que une ou plusieurs compositions sont administrées simultanément dans le même doigt et en ce que le traitement comprend un ou plusieurs intervalles pendant lesquels le traitement est délivré à partir de ces administrations ponctuelles.

Les administrations par injection sous-cutanée des implants ou fils peuvent être réalisées avec des compositions solides chargées à l'intérieur d'aiguilles creuses de plus gros diamètre que les implants ou fils. Ces administrations peuvent ainsi être réalisées avec des compositions solides fixées à l'autre extrémité d'une aiguille pleine de même diamètre que les implants ou fils à partir d'une seule aiguille.

Par rapport aux traitements oraux, un des autres intérêts de cette approche de traitements selon l'invention et notamment des traitements antifongiques est de limiter la quantité des produits actifs dans l'ensemble de l'organisme dans des zones où ils sont indésirables et de réduire aussi considérablement la quantité totale de produit utilisé pour un même traitement.

Cela permet de plus de contrôler les risques de contamination environnementale avec les molécules émergentes issues des rejets et déchets de médicaments qui constituent une menace sérieuse croissante et difficile à éviter beaucoup mieux contrôlée en amont dans l'approche selon la présente invention, avec des quantités jusqu'à 1000 voire jusqu'à 5000 fois inférieures de ces molécules utilisées pour un même traitement, 100% de la dose de médicament utile et aucun produit éliminé sans être utilisé par le patient, comme c'est souvent le cas avec les restes de comprimés ou liquides en flacon des traitements habituels.

Un des autres intérêts de l'approche du traitement selon l'invention obtenu par les compositions retard ou contrôlées qui libèrent leurs principes actifs de façon prolongée au cours du temps est de permettre d'avoir le ou les principes actifs présents en permanence à leurs sites d'action. On sait par ailleurs que certaines de ces molécules sont susceptibles de s'accumuler au niveau de l'ongle ce qui contribue à leur efficacité dans les utilisations selon l'invention et à la possibilité de réduire les doses.

L'invention a donc aussi pour objet les utilisations de ces compositions associées à d'autres traitements comme les traitements oraux, topiques ou LASER pour assurer notamment que les infections soient traitées au niveau profond de la matrice pour assurer qu'il y ait en permanence la présence in-situ du traitement nécessaire, ou encore prolonger le traitement après ces traitements pour éviter les récidives.

Un autre objet de l'invention est de proposer un traitement loco-régional externe de durée prolongée avec d'autres compositions liquides ou non-solides, retards ou contrôlées, des maladies de l'ongle par insertion entre la plaque et le lit de l'ongle lorsqu'ils sont séparés ou facilement séparables. L'expression composition non-solide inclut les compositions liquides.

Les inventeurs ont aussi découvert que ces maladies peuvent aussi être traitées de façon externe par l'insertion entre la plaque et le lit de l'ongle d'une formulation retard ou contrôlée, liquide ou non-solide, qui va épouser les volumes disponibles et ne pas créer de barrière physique solide susceptible de contrarier la repousse d'un ongle sain.

Selon les cas cette approche pourra être soit utilisée séparément soit en combinaison avec l'utilisation des compositions solides de l'invention pour un même résultat.

Ces deux formes retard interne et externe pourront éventuellement se combiner et libérer un ou plusieurs principes actifs dans une même formulation ou dans différentes formulations administrées séparément selon les besoins de chaque patient.

Au niveau du lit de l'ongle, dans une zone qui peut être séparée de la plaque cornée par l'infection de la mycose ou dans la plaque elle-même épaissie par la mycose, et qui est difficilement accessible par les traitements topiques externes, il sera possible d'associer un traitement par insertion ou infiltration d'une suspension de mêmes principes actifs dans une forme liquide ou non-solide, qui peut être polymérisable, solidifiable ou non par différents moyens (chaleur, lumière, solvants...), réalisée par exemple à partir de monomères formant un réseau gélifié dans un solvant, aqueux ou non, qui formera un hydrogel, un organogel ou tout autre gel pouvant contenir et libérer des actifs.

Un ongle infecté subit souvent ce phénomène de soulèvement et d'épaississement de la plaque cornée par rapport au lit de l'ongle, appelée onycholyse. Cette onycholyse est due à un renouvellement des cellules du lit de l'ongle trop rapide causé par une réponse inflammatoire à l'infection de la mycose. Cette onycholyse peut être avantageuse pour l'administration de formulations contenant des actifs anti-infectieux en créant un espace entre l'ongle et le lit de l'ongle pour permettre une libération plus efficace de l'actif directement dans la zone infectée, tout en restant non-invasif et donc non-douloureux.

Un exemple de polymère(s) pouvant être utilisé(s) pour contenir le ou les principe(s) actif(s) pour ce type d'administration ou insertion externe avec un effet prolongé ou retard peut faire partie de la famille des Polyhydroxyéthylméthacrylate, des hydroxyéthyl méthacrylate, des carboxyméthylcellulose, des acides hyaluroniques, ou tout autre polymère formant un hydrogel, biodégradable ou non, en formulation seul, associés entre eux ou formulés avec d'autre excipients.

Un exemple de formulations sans effet retard pour administration en topique et à travers la peau ou l'ongle pouvant être utilisés pour contenir le principe actif formant un organogel est décrit dans les brevets WO 2009/097471 et WO 2006/042059 de Mediquest Therapeutics.

Un autre exemple de polymère et formulations associées pouvant être utilisé pour contenir le ou les principe(s) actif(s) pour administration externe est décrit dans le brevet WO2016202985 de Gecko Biomédical, qui pourra avoir un rôle d'adhésif qui libèrera le principe actif au fur et à mesure de sa biodégradation. Dans ce cas, comme les biopolymères précédents, la polymérisation peut être réalisée in-situ après administration.

Un autre exemple de formulations pouvant contenir le principe actif pour administration externe est décrit dans le brevet US 2010/0048724 de Hallux. Ici la zone d'administration proposée est entre la plaque et le lit de l'ongle, mais les compositions envisagées ne sont pas retard et donc implique des administrations fréquentes et répétées qui n'apportent pas une réponse satisfaisante pour cette zone de traitement dans laquelle il est préférable de ne pas intervenir fréquemment.

Finalement, le polymère comportant le principe actif en suspension pourra être sous forme non aqueuse et non solide ni solidifiable, réalisée par exemple à partir des PLGA selon les brevets WO2004/067602, WO2005/100439, WO2006/018524, WO2007/085729, WO2008/049631, WO2009/138589, WO2012/06619 et WO2012/066194. Cette formulation qui n'est pas solide ne contient pas de solvant organique et qui ne se dessèche pas, est susceptible d'avoir un effet prolongé local sans contrarier la repousse de l'ongle. Ces suspensions et ces traitements constituent un autre objet de l'invention.

Ces nouveaux traitements retard selon l'invention ne nécessitent pas d'application quotidienne mais hebdomadaires ou mensuelles comme les nouveaux traitements injectables.

Ils peuvent être administrés aux patients au même moment et à la même fréquence espacée que les traitements internes injectables.

Le traitement interne est susceptible d'être poursuivi sur toute la durée nécessaire éventuellement en complément et en même temps que le traitement externe retard par insertion au niveau de la plaque unguéale ou au niveau du lit de l'ongle.

La présente invention a donc également pour objet une composition liquide ou non-solide pour son utilisation dans le traitement de maladies de l'ongle et/ou pour accélérer la croissance de l'ongle comprenant un ou plusieurs polymères biodégradables ou non à libération prolongée, et un ou plusieurs principes actifs caractérisée en ce qu'elle se présente sous la forme d'un dispositif destinée à être administré par insertion dans les espaces vides ou préparés entre le lit et la plaque de l'ongle et en ce qu'elle est éventuellement utilisée de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions solides telles décrites ci-dessus et comprenant un ou plusieurs polymères biodégradables.

En d'autres termes, la présente invention a donc également pour objet une composition liquide ou non-solide comprenant un ou plusieurs monomères ou polymères biodégradables ou non, à libération prolongée et un ou plusieurs antifongiques pour son utilisation dans le traitement de maladies de l'ongle par administration par insertion dans les espaces vides ou préparés entre le lit et la plaque de l'ongle, éventuellement de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions solides telles que décrites ci-dessus et comprenant un ou plusieurs polymères biodégradables.

Les polymères pour compositions non solides biodégradables utilisables par administration par insertion dans l'espace entre le lit et la plaque de l'ongle peuvent par exemple être choisis parmi les polymères décrits par exemple dans la demande de brevet international N° WO 2008/049631.

Ces polymères peuvent ainsi être choisis de préférence parmi les polymères biodégradables à très bas poids moléculaire éventuellement coiffés à une extrémité par un groupe alkyle contenant de 5 à 18 atomes de carbone à la place de son extrémité carboxylique choisis parmi les acides polylactiques, les acides polyglycoliques, les acides poly(lactide-co-glycolide), et les mélanges de ceux-ci.

Ces compositions non solides et non solidifiables comprenant donc un ou plusieurs polymères, biodégradables à libération prolongée peuvent utiliser les mêmes principes actifs que ces compositions solides de l'invention qui contiennent un polymère de préférence dans des formes à base de PLGA.

Le poids moléculaire de ces polymères ou copolymères pour compositions non solides aussi appelés semi-solides, biodégradables est compris de préférence entre 700 et 3000 Daltons, de préférence entre 800 et 2000 Daltons.

La présente invention a notamment pour objet une composition comprenant un ou plusieurs polymères non solides biodégradables telle que décrite ci-dessus caractérisée en ce que le polymère non solide à libération prolongé est choisi parmi les acides polylactiques, les acides polyglycoliques, les acides poly(lactide-co-glycolide)s, et les mélanges de ceux-ci, et en ce que le poids moléculaire de ces polymères ou copolymères est compris de préférence entre 700 et 3000 Daltons, de préférence entre 800 et 2000 Daltons.

La présente invention a également pour objet un vernis à ongles à action antimycotique comprenant un ou plusieurs antifongiques choisis de préférence parmi la Nystatine, l'Amphotéricine B, l'Abafungine, le Benzalkonium chloride, la Caspofungine, le Cetrimide, le Clioquinol, le Sulphate de cuivre, l'Haloprogine, les Echinocandines, la Flucytosine, le Mycobactovir, la Novexatine, la Natamycine, le Cetylpyridium chlorure, la Benzylamine butenafine, les Benzoxaboroles (dérivés cycliques des acides boroniques), l'Albaconazole, l'Arasertaconazole, le Bifonazole, le Butoconazole, le Clotrimazole, l'Eberconazole l'Éconazole, l'Eficonazole, le Fenticonazole, le Fluconazole, le Fosravuconazole, l'Isoconazole, l'Irtemazole, l'Isavuconazole, le Ketoconazole, le Liarozole, le Lianozole, le Luliconazole, le Miconazole, l'Oxadiazole, l'Oxiconazole, le Posaconazole, le Pramiconazole, le Ravuconazole, le Sertaconazole, le Sulconazole, le Terconazole, le Thiazole, le Thiabendazole, le Thiadiazole, le Thiamazole, le Tioconazole, le Voriconazole, l'Amiodarone, la Naftifine, le Tavaborole, la Butenafine, la Flucytosine, la Griséofulvine, la Caspofungine, la Micafungine, l'Oxide nitrique, l'Oxychlorosène de sodium, la Povidoneiodine, le Thiocarbonate tolnafate, la Sulbentine, le Zync Pyrithione et plus particulièrement parmi la Terbinafine, le Ciclopirox, la Ciclopiroxolamine, l'Itraconazole, et l'Amorolfine pour son utilisation dans le traitement de maladies de l'ongle par application sur les ongles de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions solides comprenant un ou plusieurs polymères biodégradables telles que décrites ci-dessus et/ou avec l'administration par insertion dans l'espace entre le lit et la plaque de l'ongle d'une ou plusieurs compositions non solides comprenant un ou plusieurs polymères biodégradables telle que décrite ci-dessus.

Comme indiqué ci-dessus, les vernis utilisés contiennent un ou plusieurs antifongiques qui peuvent être choisis parmi les antifongiques mentionnés ci-dessus. Ces vernis contiennent au maximum 10% en agent thérapeutique sauf le Trosyd ® de Pfizer qui peut en contenir jusqu'à 28% de principe actif.

Le vernis Penlac® de Valeant Pharmaceuticals (ciclopirox solution, 8%) qui a été approuvé par la FDA peut par exemple être utilisé.

La présente invention a également pour objet une composition pharmaceutique antifongique comprenant un ou plusieurs antifongiques choisis de préférence parmi la Nystatine, l'Amphotéricine B, l'Abafungine, le Benzalkonium chloride, la Caspofungine, le Cetrimide, le Clioquinol, le Sulphate de cuivre, l'Haloprogine, les Echinocandines, la Flucytosine, le Mycobactovir, la Novexatine, la Natamycine, le Cetylpyridium chlorure, la Benzylamine butenafine, les Benzoxaboroles (dérivés cycliques des acides boroniques), l'Albaconazole, l'Arasertaconazole, le Bifonazole, le Butoconazole, le Clotrimazole, l'Eberconazole l'Éconazole, l'Eficonazole, le Fenticonazole, le Fluconazole, le Fosravuconazole, l'Isoconazole, l'Irtemazole, l'Isavuconazole, le Ketoconazole, le Liarozole, le Lianozole, le Luliconazole, le Miconazole, l'Oxadiazole, l'Oxiconazole, le Posaconazole, le Pramiconazole, le Ravuconazole, le Sertaconazole, le Sulconazole, le Terconazole, le Thiazole, le Thiabendazole, le Thiadiazole, le Thiamazole, le Tioconazole, le Voriconazole, l'Amiodarone, la Naftifine, le Tavaborole, la Butenafine, la Flucytosine, la Griséofulvine, la Caspofungine, la Micafungine, l'Oxide nitrique, l'Oxychlorosène de sodium, la Povidoneiodine, le Thiocarbonate tolnafate, la Sulbentine, le Zync Pyrithione et plus particulièrement parmi la Terbinafine, le Ciclopirox, la Ciclopiroxolamine, l'Itraconazole, et l'Amorolfine pour son utilisation dans le traitement de maladies de l'ongle par administration par voie orale ou injectable de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions solides comprenant un ou plusieurs polymères biodégradables telles que décrites ci-dessus et/ou avec l'administration par insertion dans l'espace entre le lit et la plaque de l'ongle d'une ou plusieurs compositions polymères non solides comprenant un ou plusieurs biodégradables telle que décrite ci-dessus et/ou avec l'application sur les ongles d'un vernis à ongle à action antifongique tel que décrit ci-dessus

Les antifongiques utilisés par voie orale peuvent être choisis dans la liste mentionnée ci-dessus et de préférence parmi le kétoconazole, l'itraconazole, la terbinafine chlorhydrate, le fluconazole la griseofulvine et en cas de traitement d'urgence hospitalier le posaconazole ou le voriconazole.

Comme indiqué ci-dessus, les traitements utilisant les vernis et/ou les traitements oraux présentent des inconvénients auxquels l'administration par voie sous-cutanée à une distance supérieure à 1cm du bord de l'ongle de préférence dans le doigt à l'exception de la dernière phalange d'une ou plusieurs compositions comprenant un ou plusieurs polymères biodégradables telles que décrites ci-dessus apportent des solutions thérapeutiques très avantageuses.

Il peut cependant, dans certains cas, être intéressant de combiner ces traitements par vernis et/ou voie orale, pour une durée limitée et avec des doses éventuellement réduites avec l'administration par voie sous-cutanée à une distance supérieure à 1cm du bord de l'ongle de préférence dans le doigt à l'exception de la dernière phalange d'une ou plusieurs compositions comprenant un ou plusieurs polymères solides biodégradables telles que décrites ci-dessus.

Ces différents traitements peuvent ainsi être combinés dans le but d'en renforcer (booster) l'efficacité. L'utilisation des compositions comprenant un ou plusieurs polymères solides biodégradables à libération prolongée, et éventuellement un ou plusieurs principes actifs peut ainsi par exemple s'ajouter à un traitement par voie orale telle que décrit ci-dessus.

L'invention a donc aussi pour objet une composition pour son utilisation dans le traitement des mycoses de l'ongle caractérisée en ce qu'elle comprend un ou plusieurs polymères choisis parmi les PLA et les PGLA, entre 0,04 et 6.5mg de chlorhydrate de terbinafine, de ciclopirox ou de ciclopiroxolamine et en ce qu'elle se présente sous la forme d'un implant d'une longueur comprise entre 4 et 15mm et un diamètre compris entre 0,3 et 0,8 mm destiné à être administré par voie sous-cutanée à une distance supérieure à 1cm du bord de l'ongle dans le doigt à l'exception de la dernière phalange.

La présente invention a également pour objet une composition telle que décrite ci-dessus caractérisée en ce qu'elle se présente de préférence sous la forme d'un implant présentant éventuellement une extrémité aiguisée, d'un tube cylindrique ou d'un fil et en ce que la composition est administrée dans le doigt à l'exception de la dernière phalange pour son utilisation dans le traitement de maladies de l'ongle par administration de façon simultanée ou étalée dans le temps avec :
- l'administration par insertion dans l'espace entre le lit et la plaque de l'ongle d'une composition non solide comprenant un ou plusieurs polymères biodégradables à libération prolongée, et éventuellement un ou plusieurs principes actifs de préférence un ou plusieurs antifongiques
   et/ou
- l'application sur les ongles d'un vernis à ongles à action antimycotique comprenant un ou plusieurs antifongiques choisis de préférence parmi le fluconazole, le kétoconazole, le miconazole, la terbinafine, le tolnaftate,l'amorolfine, le ciclopirox, ou le miconazole,
   et/ou
- l'administration par voie orale ou injectable d'une composition pharmaceutique antifongique comprenant un ou plusieurs antifongiques choisis de préférence parmi les dérivés azotés tels que le kétoconazole, le fluconazole, l'isavuconazole, l'traconazole, le posaconazole ou le voriconazole; les échinocandines telles que l'anidulafungine, la caspofungine, la micafungine, la flucytosine, la griséofulvine et la terbinafine; l'amphotéricine B
   et/ou
- un traitement par LASER provoquant la destruction du champignon par effet thermique photobiologique.
L'utilisation d'un faisceau LASER dans le but de détruire les champignons est connue de l'homme de métier et est décrite ci-dessus.

La demanderesse a également découvert des conditions de fabrication, selon un procédé avantageux, permettant l'obtention de certaines desdites compositions selon l'invention. En effet, une des contraintes de l'administration loco-régionales des compositions selon l'invention est que la zone d'administration est un espace restreint et donc limite fortement la quantité de formulation pouvant être administrée. Par ailleurs, il est donc très important de pouvoir y administrer des formulations à haute teneur en principes actifs, ce qui est antinomique avec la nécessité d'ajouter aux substances actives des ingrédients pharmaceutiques aidant à contrôler la libération lente des actifs en quantité suffisante pour une action de longue durée, et ainsi éviter d'avoir à répéter les administrations à une fréquence trop rapprochée, inférieure à un mois, voire une semaine.

Il est par ailleurs évident pour l'homme de l'art que plus on augmente la teneur en principe actif (ou core loading en anglais) dans des compositions pharmaceutiques à libération prolongé, plus il est difficile d'obtenir un relargage contrôlé et donc une durée d'action prolongée.

De façon surprenante et non attendue, une solution trouvée dans notre nouvelle composition est d'associer la substance active sous la forme de particules en quantité importante et de les maintenir associées mais isolées dans le processus de préparation par une faible quantité de polymère qui va pouvoir recouvrir ses particules pour les isoler même avec un pourcentage bas de ce polymère dans le mélange.

On a observé que dans certaines conditions de fabrication, il était possible d'obtenir une telle formulation sous la forme solide d'un implant fragile ou cassant constitué desdites particules de substances entourées de polymère dont l'aspect à la surface est donc rugueux, filandreux ou non lisse et dont la section laisse apparaître cet assemblage de la substance active sous forme de particules transparentes, cristallines ou vitreuses entourées de polymère opaque, blanc et d'aspect filandreux.

De façon surprenante/inattendue cette assemblage permet d'obtenir un effet retard contrôlé et prolongé, par exemple sur plusieurs mois au fur et à mesure que les particules de substance actives seront exposées aux tissus biologiques ou au milieu environnant au site d'administration pour que le produit puisse ensuite être libéré lorsque le polymère va se biorésorber.

Par ailleurs, la surface non lisse biocompatible et biorésorbable des compositions obtenues permet une bonne adhérence au tissu sous-cutané ou au milieu environnant, les irrégularités multipliant ainsi la surface de contact avec le tissu lors de l'implantation ou de l'insertion et assure ainsi le maintien en locorégional à l'emplacement souhaité.

Les compositions selon l'invention peuvent être préparées par les techniques connues de l'homme de l'art pour l'obtention de formes pharmaceutiques parentérales stériles contenant des polymères biodégradables.

De préférence, sans que cela soit limitatif, les différents constituants des compositions peuvent être, après pesée, tamisage et mélange pour les formes non solides, soumis à un procédé d'extrusion fusion, injection moulage, d'impression 3D ou de compactage pour obtenir la forme finale des formes solides à la géométrie et effet prolongé désiré.

De préférence, lors d'un procédé de fusion-extrusion la température sera choisie de façon avantageuse pour faire fondre le polymère ou le mélange de polymères autour des substances actives solides et non fondues, permettant ainsi un recouvrement partiel ou total de la substance active pour obtenir l'effet de libération prolongé désiré. Cette opération de fusion a lieu en même temps que l'opération d'extrusion, avec ou sans étirement pour permettre ce filage des différents composants.

Le choix des conditions opératoires associées à la composition permet de contrôler l'organisation intérieure et extérieure des différents composants et ainsi assurer son activité.

De façon surprenante sans qu'il n'y ait d'opération de prétraitement avec des solvants ou séchage des particules solides avant leur mélange, les mélanges de compositions selon l'invention permettent d'obtenir des compositions homogènes.

Suivant le grade et le fabricant de polymères, celui-ci est fourni sous forme solide de pellets granulé, « filaments », ou poudres prêtes à l'emploi, obtenues dans la majorité des cas par cryobroyage des polymères.

Dans le cas où le polymère ne serait pas fourni à l'état de poudre pour son mélange direct avec la ou les substances actives et autres composants, une opération destinée à réduire leur densité apparente et faciliter le mélange des poudres avant mise en forme peut être avantageusement appliquée. A titre d'exemple non limitatif, l'homme de l'art peut choisir de cryobroyer le polymère, le compacter et le granuler à sec à une température inférieure ou égale à 25 °C voire procéder à une granulation en solvant organique ou une étape d'extrusion broyage.

Une fois le polymère réduit à l'état de poudre et mélangé ou non aux autres composants, le mélange est introduit dans la cavité de l'extrudeuse où il est ensuite amené à sa température de fusion directement et rapidement au cours du procédé d'extrusion.

Le mélange des substances actives avec le polymère ou copolymère d'un acide lactique et/ou d'acide glycolique ou le mélange de polymères et/ou copolymères d'acide lactique et/ou d'acide glycolique forme une pâte de mélange fondue.

Ledit mélange est ainsi alimenté dans une vis d'extrusion selon un procédé tel que le temps de liquéfaction-fusion du mélange et de transit jusqu'à la buse d'extrusion soit réduit et inférieur à 30 minutes et préférentiellement inférieur à 15 minutes.

Selon cette variante avantageuse du procédé de fabrication, il est possible d'opérer sans prétraitement du mélange, donc sans solvants aqueux ou organiques et/ou sans lyophilisation des mélanges et sans préchauffage distinct pour la compression avant extrusion, ce qui permet de contrôler le cas échéant le faible état d'hydratation dudit mélange et d'extruder à des températures pouvant être supérieures à 100 °C, de préférence comprises entre 110 et 160 °C, et plus préférentiellement entre 125 °C et 150 °C voire entre 137 et 145 °C, par exemple à environ 143 °C, sans dégradation du principe actif, sur des temps de chauffage courts, inférieurs à 15 minutes, de préférence compris entre 5 et 10 minutes.

Dans le cas de l'extrusion, cet arrangement à chaud pourra donner la forme souhaitée directement grâce au mélange de la vis et au diamètre de la buse d'extrusion. II sera également possible d'effectuer un contrôle de la forme solide et notamment de son diamètre grâce à une étireuse régulant le diamètre de l'extrudât.

Dans ce cas et selon le diamètre souhaité, l'étireuse pourra agir à température ambiante à la sortie de l'extrudeuse. L'extrudât pourra également passer à travers une chambre thermostatée à température élevée, égale ou inférieure à la température d'extrusion pour permettre un étirage plus important et notamment l'obtention de très petits diamètres (par exemple inférieurs à 0,1 mm ou encore inférieur à 0,05 mm).

Cet extrudât continu pourra ensuite être coupé à la taille (surface d'échange) offrant le profil de relargage souhaité, par exemple par cryobroyage ou à l'aide d'une guillotine au sortir d'extrusion.

Selon la forme, la dose et le profil de relargage souhaité, ce procédé de fabrication pourra aussi s'appliquer à des formes de faibles charges en principe actif, inférieures à 20 % notamment comprises entre 0.5 et 10 % ou à de fortes charges, supérieures à 50 % et notamment comprises entre 60 et 80 %.

L'implant peut alors être placé dans un dispositif d'injection et irradié par gamma-irradiation avant administration.

Le dispositif d'injection peut être prérempli et déjà contenir l'implant, ou peut être chargé juste avant administration. De même, lorsqu'il est nécessaire d'injecter plusieurs implants par doigt/orteil ou par personne, il peut être intéressant au niveau packaging et économique d'utiliser un dispositif réutilisable. Dans ce cas, l'implant est contenu directement dans l'aiguille servant à l'administration qui peut être changée lors de chaque administration.

Dans le cas où le dispositif n'est pas pensé pour être réutilisé, il peut être pourvu d'un système de blocage pour empêcher de pouvoir s'en servir à nouveau. Ce système peut aussi cacher l'aiguille à la vue de l'utilisateur pour réduire les possibles risques de blessures par piqûre d'aiguille.

### PARTIE EXPERIMENTALE

### I - PROCEDURES GENERALES

### A - PROCEDURE GENERALE DE PREPARATION DES COMPOSITION DES EXEMPLES

Les compositions selon l'invention présentées dans les exemples ci-dessous sont obtenues à partir de procédés pharmaceutiques connus par l'homme de l'art.

En fonction de l'usage fait des différentes compositions, les tailles de lots peuvent varier de quelques milligrammes à plusieurs centaines de grammes, voire kilogrammes.

Le polymère biodégradable, mélange de polymères ou (co)polymères utilisés dans les exemples suivants sont avantageusement préalablement tamisés sur une maille de 600 micromètres ou broyés dans un moulin cryogénique (cyogenic grinder) de type SPEX 6775 Freezer/Mill ®.

A la fin du protocole, une poudre fine et homogène est récupérée et peut être utilisée dans les différentes procédures de préparation.

Par la suite, le polymère ou mélange de (co)polymères, le principe actif, les différents excipients ou mélanges de principe actifs et excipients sont pesés séparément.

Les composants sont ensuite mélangés à l'aide d'un mélangeur par retournement (mélangeur agitateur ou stirrer) type Turbula® Type T 2F à une vitesse de 49 tours.min⁻¹ pendant 10 minutes.

Les pourcentages (%) dans les exemples suivants sont exprimés en masse (m/m).

### (1) Extrusion

Après mélange des poudres constituant les compositions, le mélange est ensuite soumis à un processus de fusion-extrusion en continu sur une extrudeuse monovis équipée d'une vis sans fin de pas de 8mm, de contrôle de pressions, d'une filière ou buse de diamètre variable et d'un système d'étirement en continu en sortie de filière pour réguler le diamètre des implants. La température lors de ce processus est adaptée en fonction des compositions et principes actifs et est détaillée dans chaque exemple à suivre. L'étirement peut aussi être régulé en fonction du diamètre final désiré.

Après analyse chimique du contenu en principe actif et pureté, l'implant est coupé à la longueur souhaitée et stérilisé par gamma-irradiation à 25 kGy.

### (2) Compression

Dans ce procédé de fabrication, afin de réduire le volume apparent du polymère utilisé, une première étape de granulation par voie sèche est effectuée. Les composants sont ensuite mélangés et servent à alimenter la trémie de la machine à comprimer.

Le polymère ou mélange de (co)polymères, le principe actif ou mélanges de principe actifs sont comprimés à l'aide d'une presse, par exemple une machine à comprimer de type Korsch PH106DMS rotative.

En fonction de la taille et de la forme du poinçon utilisé durant la compression, il est possible d'obtenir des implants de dimensions, charge en principe actif et masse variables.

Les implants obtenus sont ensuite stérilisés par gamma-irradiation à 25 kGy

### (3) Impression 3D

L'extrudat obtenu par extrusion selon le procédé décrit ci-dessus en (1) peut être souple et donc enroulé comme un fil ou filament. Il est possible de fabriquer un extrudat de diamètre 1,75 mm pour pouvoir être utilisé avec une imprimante 3D de type FDP (Fusion Deposition Modeling en anglais), comme par exemple la PRUSA i3 HD, avec les caractéristiques suivantes :
- Diamètre du filament : 1.75mm
- Hauteur de couche extrudée : 0.10mm
- Extruder hotend pack V5: PEEK-brass nozzle, 0.35mm de diamètre
- Electronique : ARDUINO MEGA 2560* RAMPS v1.4
- Plateforme : lit en verre avec régulation de température

Une fois imprimés et refroidis, les implants sont récupérés avec une pince sur la plateforme en verre avant analyse. Les implants obtenus sont ensuite stérilisés par gamma-irradiation à 25 kGy

### B- PROCEDURE GENERALE D'ANALYSE DES IMPLANTS

Dans les procédures exposées ci-dessous en B (1) à (6), les analyses sont réalisées sur un chromatographe en phase liquide à haute performance en phase inverse (RP-HPLC), tels que les systèmes Alliance de la société Waters. La détection est réalisée par un détecteur UV/Visible, comme par exemple le détecteur UV/Visible 2489 de Waters à une longueur d'onde variable selon le principe actif.

Le chromatographe est équipé d'une pré-colonne de type Waters X Bridge C18, 20x4,6 mm, 5µm et d'une colonne de type Waters X Bridge C18, 150x4,6mm, 5µm. La température de colonne est maintenue à 35°C.

### (1) Méthode RP-HPLC pour déterminer la pureté, identifier et quantifier la Terbinafine

### PREPARATION DES ECHANTILLONS

Blanc : Acétonitrile/Eau Milli-Q (25/75, v/v)
Les standards sont préparés à une concentration de 30µg/mL de terbinafine dans une solution acétonitrile/eau MilliQ (25/75, v/v)
Les échantillons à analyser sont solubilisés puis dilués à une concentration approximative de 30µg/mL de terbinafine dans une solution acétonitrile/Eau Milli-Q (25/75, v/v).

### CONDITIONS DE CHROMATOGRAPHIE HPLC

Un gradient d'élution est appliqué à partir de 2 phases mobiles :

| | |
|---|---|
| Phase mobile A: | THF/ACN/Citrate Buffer pH4.5 (10/20/70) (v/v/v) |
| Phase mobile B: | Acétonitrile |

| t(min) | A (%) | B (%) |
|---|---|---|
| 0 | 80.0 | 20.0 |
| 25.0 | 55.0 | 45.0 |
| 26.0 | 80.0 | 20.0 |
| 31.0 | 80.0 | 20.0 |

| | |
|---|---|
| -Flux: | 0.9 mL/min |
| -Volume injection: | 30µL |
| -Temps d'analyse: | 32 min. |

La détection du pic principale de terbinafine se fait à la longueur d'onde de 280nm / 226nm. Pour quantifier et évaluer la pureté, seule la longueur d'onde de 226nm est utilisée.

Les résultats de contenu en actif et de pureté sont déterminés à partir du chromatogramme du standard de référence et du chromatogramme de la terbinafine à un temps de rétention approximatif de 13,5 min.

### (2) Méthode simplifiée pour la quantification de la Terbinafine

### TEST IN VITRO

Afin d'évaluer les propriétés de libération du principe actif contenu dans l'implant, un test de libération *In Vitro* est réalisé à 37°C pendant 7 jours puis la température est augmentée à 55°C au bout de 7 jours dans un milieu physiologiquement acceptable (0.9% NaCl). Un minimum de 3 échantillons individuels provenant du même lot est testé.

Les implants préalablement pesés sont introduits directement dans des réservoirs contenant 5mL du milieu physiologique. 100µL de la solution sont extraits à des temps prédéterminés et stockés à température ambiante jusqu'à analyse.
Les temps prédéterminés sont les suivants : t=0, t=1h, t=5h, t=1j, t=2j, t=4j, t=7j, t=8j, t=9j et t= 15j.

### PREPARATION DES ECHANTILLONS

Les échantillons sont directement analysés ou peuvent être dilués à priori en fonction de la concentration (dilution effectuée avec une solution acétonitrile/eau Milli-Q (25:75, v/v))
Blanc: acétonitrile/Eau Milli-Q (25:75, v/v)
Les standards sont préparés à une concentration de 30µg/mL de terbinafine dans un mélange acétonitrile/eau Milli-Q (25:75, v/v)

### CONDITIONS DE CHROMATOGRAPHIE HPLC

Un gradient d'élution est appliqué à partir de 2 phases mobiles :

| | |
|---|---|
| Phase mobile A: | THF/ACN/Citrate Buffer pH4.5 (10/20/70) (v/v/v) |
| Phase mobile B: | Acétonitrile/Eau Milli-Q (95/5, v/v) |

| t (min) | A (%) | B (%) |
|---|---|---|
| 0 | 80.0 | 20.0 |
| 4.0 | 10.0 | 90.0 |
| 6.0 | 80.0 | 20.0 |
| 10.0 | 80.0 | 20.0 |

| | |
|---|---|
| -Flux: | 0.9 mL/min |
| -Volume injecté: | 30µL |
| -Temps d'analyse: | 10 min. |

La détection du pic principale de terbinafine se fait à la longueur d'onde de 280nm.
Les résultats de contenu en principe actif et de pureté sont déterminés à partir du chromatogramme du standard de référence et du chromatogramme de la terbinafine à un temps de rétention approximatif de 6,4 min.

### (3) Méthode pour déterminer la pureté, identifier et quantifier le Ciclopirox

### PREPARATION DES ECHANTILLONS

Blanc: Acétonitrile/eau Milli-Q (15/85, v/v)
Les standards sont préparés à une concentration de 25µg/mL de ciclopirox dans une solution acétonitrile/eau Milli-Q (15/85, v/v)
Les échantillons sont dilués à une concentration approximative de 25µg/mL de ciclopirox dans une solution acétonitrile/eau Milli-Q (15/85, v/v).

Les échantillons, blancs et standards sont préparés selon le processus suivant (avant injection dans le système HPLC) :
- 800µL d'échantillon
- 400µL de NaOH 0.1M
- 80µL de Diméthyl Sulfate
- Mélange au Vortex (agitateur) (15 secondes)
- Incubation à 37°C (15 minutes)
- 80µL de Triéthylamine
- Mélange au Vortex (15 secondes)

### CONDITIONS CHROMATOGRAPHIE HPLC

Un gradient d'élution est appliqué à partir de 2 phases mobiles :

| | |
|---|---|
| Phase mobile A: | Eau Milli-Q |
| Phase mobile B: | Acétonitrile |

| t (min) | A (%) | B (%) |
|---|---|---|
| 0 | 85 | 15 |
| 25 | 50 | 50 |
| 26 | 20 | 80 |
| 27 | 85 | 15 |
| 32 | 85 | 15 |

| | |
|---|---|
| -Flux: | 1.0 mL/min |
| -Volume injecté: | 60µL |
| -Temps d'analyse: | 32 min |

La détection du pic principale de ciclopirox a lieu à la longueur d'onde de : UV @ 305nm. Les résultats de contenu en actif et de pureté sont déterminés à partir du chromatogramme du standard de référence et du chromatogramme du ciclopirox à un temps de rétention approximatif de 20,2 min.

### (4) Méthode simplifiée pour la quantification du Ciclopirox

### TEST IN VITRO

Afin d'évaluer les propriétés de libération du principe actif contenu dans l'implant, un test de libération *In Vitro* est réalisé à 37°C pendant 7 jours dans un milieu physiologiquement acceptable (PBS pH 7.4). Un minimum de 3 échantillons individuels provenant du même lot est testé.

Les implants préalablement pesés sont introduits directement dans réservoirs contenant 15mL du milieu physiologique. 100µL de la solution sont extraits à des temps pré-déterminés et stockés à 5°C jusqu'à analyse.
Les temps pré-déterminés sont les suivants : t=0, t=1h, t=5h, t=1j, t=2j, t=4j et t=7j.

### PREPARATION DES ECHANTILLONS

Blanc: Acétonitrile/eau Milli-Q (15/85, v/v)
Les standards sont préparés à une concentration de 25µg/mL de ciclopirox dans une solution acétonitrile/eau Milli-Q (15/85, v/v)
Les échantillons sont injectés directement des tests *In Vitro.*

Les échantillons, blancs et standards sont préparés selon le processus suivant (avant injection dans le système HPLC) :
- 800µL d'échantillon
- 400µL de NaOH 0.1M
- 80µL de Diméthyl Sulfate
- Mélange au Vortex (à peu près 15 secondes)
- Mélange au Vortex (15 secondes)
- 80µL de Triéthylamine
- Mélange au Vortex (15 secondes)

### CONDITIONS CHROMATOGRAPHIE HPLC

Un gradient d'élution est appliqué à partir de 2 phases mobiles :

| | |
|---|---|
| Phase mobile A: | Eau Milli-Q |
| Phase mobile B: | Acétonitrile |

| T (min) | A (%) | B (%) |
|---|---|---|
| 0 | 85 | 15 |
| 4 | 0 | 100 |
| 5 | 0 | 100 |
| 6 | 85 | 15 |
| 9 | 85 | 15 |

| | |
|---|---|
| -Flux: | 1,0 mL/min |
| -Volume injecté: | 60µL |
| -Temps d'analyse: | 9 min. |

La détection du pic principale de ciclopirox se fait à la longueur d'onde de 305nm.

Les résultats de contenu en actif et de pureté sont déterminés à partir du chromatogramme du standard de référence et du chromatogramme du ciclopirox à un temps de rétention approximatif de 5,3 min.

### (5) Méthode RP-HPLC pour déterminer la pureté, identifier et quantifier l'Amorolfine

### TEST IN VITRO

Afin d'évaluer les propriétés de libération du principe actif contenu dans l'implant, un test de libération *In Vitro* est réalisé à 37°C pendant 7 jours puis est augmenté à 55°C au bout de 7 jours dans un milieu physiologiquement acceptable (0,9% NaCl). Un minimum de 3 échantillons individuels provenant du même lot est testé.

Les implants préalablement pesés sont introduits directement dans réservoirs contenant 5mL du milieu physiologique. 100µL de la solution sont extraits à des temps prédéterminés et stockés à température ambiante jusqu'à analyse. Le milieu n'est pas rempli à nouveau après extraction du volume.
Les temps prédéterminés sont les suivants : t=0, t=1h, t=5h, t=1j, t=2j, t=4j, t=7j, t=8j, t=9j et t= 15j.

### PREPARATION DES ECHANTILLONS

Blanc: Acétonitrile/eau Milli-Q (25/75, v/v)
Les standards sont préparés à une concentration de 200µg/mL d'amorolfine dans une solution acétonitrile/eau Milli-Q (25/75, v/v)
Les échantillons sont dilués à une concentration approximative de 200µg/mL d'amorolfine dans une solution d'acétonitrile/eau Milli-Q (25/75, v/v)

### CONDITIONS DE CHROMATOGRAPHIE HPLC

Un gradient d'élution est appliqué à partir de 2 phases mobiles :

| | |
|---|---|
| Phase mobile A: | THF/ACN/Citrate Buffer pH4.5 (10/20/70) (v/v/v) |
| Phase mobile B: | Acétonitrile |

| t (min) | A (%) | B (%) |
|---|---|---|
| 0 | 80 | 20 |
| 25 | 55 | 45 |
| 26 | 80 | 20 |
| 31 | 80 | 20 |

| | |
|---|---|
| -Flux: | 0,9 mL/min |
| -Volume injecté: | 30µL |
| -Temps d'analyse : | 32 min |

La détection du pic principale d'amorolfine se fait à longueur d'onde de 224/265nm. Pour quantifier et évaluer la pureté, seule la longueur d'onde de 265nm est utilisée.

Les résultats de contenu en actif et de pureté sont déterminés à partir du chromatogramme du standard de référence et du chromatogramme d'amorolfine à un temps de rétention approximatif de 12,6min.

### (6) Méthode RP-HPLC pour déterminer la pureté, identifier et quantifier l'Itraconazole

### IN VITRO

Afin d'évaluer les propriétés de libération du principe actif contenu dans l'implant, un test de libération *In Vitro* est réalisé à 37°C pendant 7 jours puis la température est augmentée à 55°C au bout de 7 jours dans un milieu physiologiquement acceptable (0.9% NaCl). Un minimum de 3 échantillons individuels provenant du même lot est testé.

Les implants préalablement pesés sont introduits directement dans réservoirs contenant 5mL du milieu physiologique. 100µL de la solution sont extraits à des temps prédéterminés et stockés à température ambiante jusqu'à analyse. Le milieu n'est pas rempli à nouveau après extraction du volume.
Les temps prédéterminés sont les suivants : t=0, t=1h, t=5h, t=1j, t=2j, t=4j, t=7j, t=8j, t=9j et t= 15j.

### PREPARATION DES ECHANTILLONS

Blanc: THF/ACN/Phase mobile A (15/10/75) (v/v/v)
Les standards sont préparés à une concentration de 50µg/mL d'itraconazole dans une solution THF/ACN/Phase mobile A (15/10/75) (v/v/v)
Les échantillons sont dilués à une concentration approximative de 50µg/mL d'itraconazole avec une solution THF/ACN/Phase mobile A (15/10/75) (v/v/v)

### CONDITION DE CHROMATOGRAPHIE HPLC

Un gradient d'élution est appliqué à partir de 2 phases mobiles :

| | |
|---|---|
| Phase mobile A: | THF/ACN/Buffer Citrate pH 4.5 (10/20/70) (v/v/v) |
| Phase mobile B: | Acétonitrile |

| t (min) | A (%) | B (%) |
|---|---|---|
| 0 | 80 | 20 |
| 25 | 55 | 45 |
| 26 | 80 | 20 |
| 31 | 80 | 20 |

| | |
|---|---|
| -Flux : | 0,9 mL/min |
| -Volume injecté : | 25 µL |
| -Temps d'analyse : | 32 min |

La détection du pic principale d'itraconazole se fait à la longueur d'onde de 265nm.

Les résultats de contenu en actif et de pureté sont déterminés à partir du chromatogramme du standard de référence et du chromatogramme d'itraconazole à un temps de rétention approximatif de 19,0 min.

### C -EVALUATION DE L'ACTIVITE ANTIFONGIQUE IN VITRO D'UNE COMPOSITION SELON L'INVENTION

L'activité antifongique au fil du temps (ou capacité d'inhibition) des compositions décrites dans les Exemples 1, 2, 6, 8, 10, 12, 18 et 19 ci-après a été évaluée au moyen du test de diffusion de la composition dans l'agar (inhibition de la croissance en boîte de Pétri), réalisés sur des semences de souches Trichophyton Rubrum isolées, de Candida Krusei et/ou Candida Parapsilosis (échantillons cliniques humains d'Onychomycose). L'agent antifongique libéré par la composition dans le milieu de culture inhibe plus ou moins le développement de l'inoculum ; cette donnée est ensuite comparée à la CMI correspondante.

Préalablement au test de diffusion, la Concentration Minimale Inhibitrice (CMI) est réalisée par micro-dilution dans un milieu liquide en suivant le protocole standard CLSI M38-A2 pour connaitre la sensibilité à l'agent fongique utilisée dans le test à l'Agar. Dans ce protocole, la CMI de l'agent antifongique correspond à 80% d'inhibition (concernant le contrôle de croissance de la souche fongique).

Méthode : Un échantillon de la composition décrite dans les Exemples 1, 2, 6, 8, 10, 12, 18 et 19 est placé au centre d'une plaque de Pétri (Müller-Hinton modifié) et incubé. La variation de la croissance de la zone d'inhibition (diamètre en mm) est mesurée à des temps prédéterminés (en jours).

Cette expérience est réalisée selon le protocole standard CLSI M44-A2 - en milieu modifié Müller Hinton.

L'incubation des plaques est réalisée dans une chambre humide à deux températures d'incubation en parallèle (27°C et/ou 35 °C).

### D -PROCEDURES DE STABILITÉ

Des implants fabriqués selon l'Exemple 1 et l'Exemple 18 gamma-irradiés ont été mis en stabilité dans les conditions ICH de stabilité à différentes conditions choisies parmi 5°C, 25°C/60% d'humidité relative et 40°C/75% d'humidité relative.

La dose et la pureté de l'actif sont évaluées selon la méthode décrite ci-dessus en B à différents temps de référence.

### E - PROCEDURES DE TESTS IN VIVO

Des implants fabriqués selon l'Exemple 1, l'Exemple 3 et l'Exemple 18 ont été implantés en sous-cutané chez des rats males sprague-dawley.

L'implantation se fait avec une seringue Hypak® de 1ml (Becton Dickinson) préchargée équipée d'une aiguille 20G Henke Sass Wolf (0.9mm diamètre extérieur, 0.6mm diamètre intérieur, 16mm de longueur).

A des temps correspondants à 7 jours, 28 jours, 56 jours, 91 jours et 112 jours pour les implants décrits dans l'Exemple 1, 24 jours et 84 jours pour les implants décrits dans l'Exemple 3 et 7 jours, 30 jours et 84 jours pour les implants décrits dans l'Exemple 18 après l'implantation des implants, les implants ont été extraits des tissus sous cutanés animaux et le contenu en principe actif analysé par RP HPLC- UV selon la méthode décrite ci-dessus en B (1) à B (4).

### II - EXEMPLES DÉTAILLÉS

### Exemple 1

4 lots d'implants contenant de la Terbinafine à différentes concentrations dans du PLGA 85:15 sont préparés selon le procédé décrit ci-dessus en A (1).

Les conditions d'extrusion sont les suivantes :

| Lots | Diamètre Vis (mm) | Vitesse extrusion (rpm) | Température d'extrusion (T°C) | Diamètre de buse (mm) | Pression en sortie extrusion |
|---|---|---|---|---|---|
| (1) | 8 | 6 | 155-162 | 0,5 | N/A |
| (2) | 8 | 10 | 150-170 | 0,85 | 500-1000 (mPa) |
| (3) | 8 | 10-13 | 164 | 0,5 | 90-140 bar |
| (4) | 8 | 10-13 | 166 | 0,5 | 200-250 (mPa) |

Des implants de 10mm de longueur et 0,56mm de diamètre sont découpés.

Les lots sont ensuite évalués analytiquement selon la méthode décrite ci-dessus en B (1) et/ou B (2).

| Lots | Diamètre de l'implant (mm) | Contenu en API (%m/m) | Pureté actif % | Dose de principe actif/implant (mg) |
|---|---|---|---|---|
| (1) | 0,56mm | 4,50 | 98,97 | 0,15 |
| (2) | 0.30mm | 15,70 | 98,10 | 0,13 |
| (3) | 0.40 | 29,90 | 99,51 | 0,44 |
| (4) | 0.58 | 58,20 | 99,59 | 1,61 |

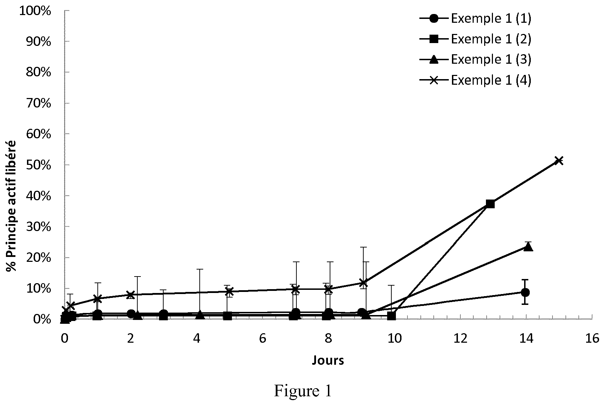

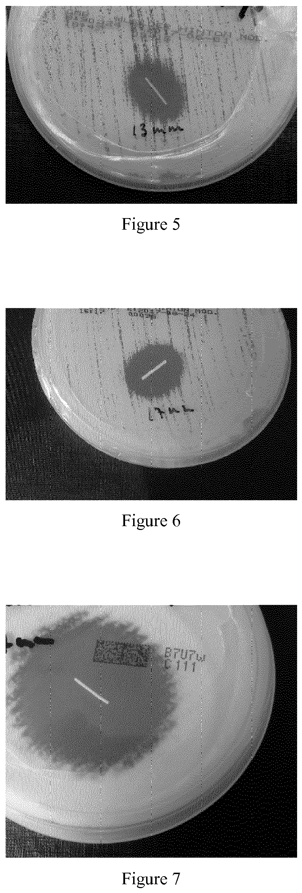

Le test de libération *In Vitro* est réalisé tel que décrit ci-dessus en B (2). Les résultats sont représentés sur la Figure 1.
L'évaluation de l'activité antifongique *In Vitro* est réalisée tel que décrit ci-dessus en C. La Figure 5 représente la photo d'une plaque d'agar après implantation d'une composition selon l'Exemple 1 (1) à 35°C dans C. Parapsilosis.

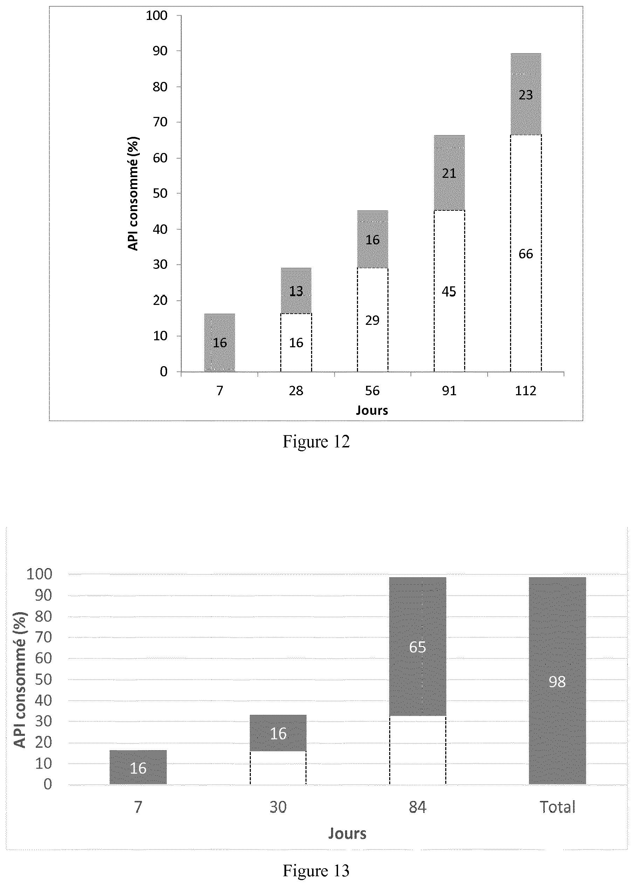

Le test *In Vivo* est réalisé tel que décrit ci-dessus en E. Les résultats du test *In Vivo* sont représentés sur la Figure 12.

La stabilité du lot (3) est déterminée selon la méthode décrite ci-dessus en D à 5°C, 25°C/60% d'humidité relative et 40°C/75% d'humidité relative, à 1 mois, 3 mois et 6 mois. Les résultats de contenu en terbinafine et de pureté et de dose d'actif obtenus sont les suivants :

| **Lot** | **Paramètre** | **Conditions** | **T0** | **T1M** | **T3M** | **T6M** |
|---|---|---|---|---|---|---|
| **Exemple 1 (3)** | **Contenu en API (%m/m)** | 5°C | 29.9 | 27.5 | 30.0 | 30.8 |
| | | 25°C / 60%RH | | 27.9 | 30.3 | 30.8 |
| | | 40°C / 75%RH | | 28.9 | NA | NA |
| | **Pureté (%Ar)** | 5°C | 99.5 | 99.4 | 99.4 | 99.4 |
| | | 25°C / 60%RH | | 99.5 | 99.4 | 99.4 |
| | | 40°C / 75%RH | | 99.5 | NA | NA |

La stabilité du lot (4) est déterminée selon la méthode décrite ci-dessus en D à 5°C, à 9 mois et 12mois. Les résultats de contenu en terbinafine, de pureté et de dose d'actif obtenus sont les suivants :

| **Lot** | **Paramètre** | **T0** | **T9M** | **T12M** |
|---|---|---|---|---|
| | | **5°C** | **5°C** | **5°C** |
| **Exemple 1(4)** | **Content (%, w/w)** | 58.2 | 58.5 | 57.9 |
| | **Dose (mg)** | 1.61 | 1.58 | 1.54 |
| | **Purity (%Ar)** | 99.6 | 99.6 | 99.3 |

### Exemple 2

Un lot d'implants contenant de la Terbinafine et du PLGA 85:15 est préparé selon le procédé décrit ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 3.5-7 | 166 | 0,85 | 2500 |

Des implants de 25mm de longueur et 0,80mm de diamètre sont découpés.

| Contenu en API (%m/m) | Pureté actif % | Dose de principe actif /implant (mg) |
|---|---|---|
| 57,70 | 99,80 | 5,70 |

Des implants de 10mm de longueur et 0,80mm de diamètre sont découpés.

| Contenu en API (%m/m) | Pureté actif % | Dose de principe actif /implant (mg) |
|---|---|---|
| 57,70 | 99,80 | 2.28 |

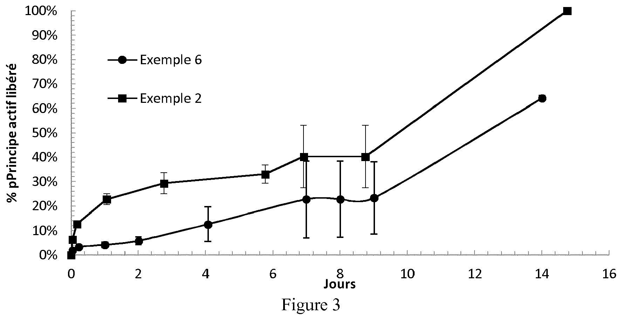

Le test de libération *In Vitro* est réalisé avec les implants de 10mm de longueur tels que décrits ci-dessus en B (2). Les résultats sont représentés sur la Figure 3.

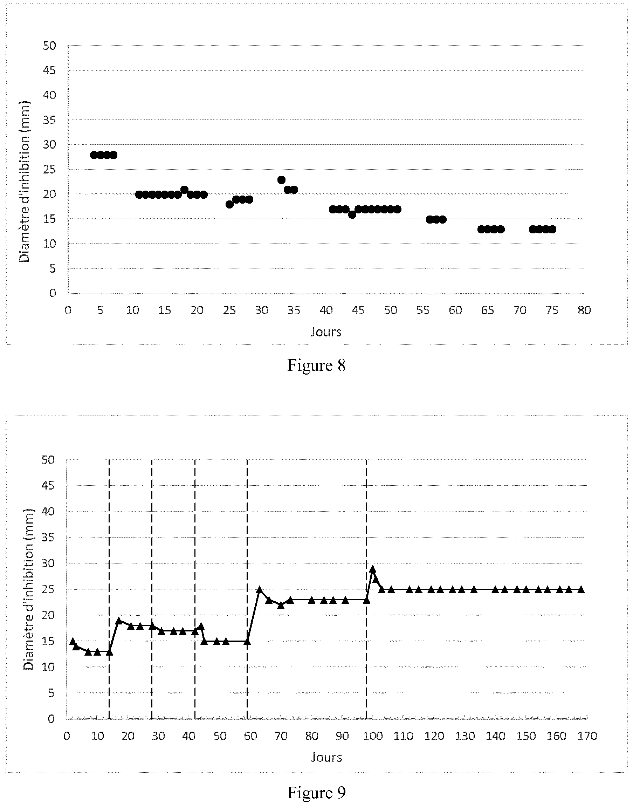

L'évaluation de l'activité antifongique *In Vitro* est réalisée selon la méthode décrite ci-dessus en C. La Figure 6 représente la photo d'une plaque d'agar après implantation d'un composé selon l'Exemple 2 dans Candidae Parapsilosis. Les résultats sont résumés sur la Figure 9.

### Exemple 3

Un lot d'implants contenant de la dexaméthasone et PLGA 75:25 est préparé selon le procédé décrit ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 5 | 130 | 0,85 | 1500-2000 |

Des implants de 6mm de longueur et 0,45mm de diamètre sont découpés.

| Contenu en API % m/m | Pureté actif % | Dose de principe actif /implant (mg) |
|---|---|---|
| 55,0 | 99,15 | 0,646 |

Le test *In Vivo* est réalisé tel que décrit ci-dessus en E.

### Exemple 4

Un lot d'implants contenant de la cyclopiroxolamine à 60% et du PLGA 85:15 à 40% est préparé selon le procédé décrit ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 6 | 155 | 0,85 | 2000 |

Des implants de 10mm de longueur et 0,60mm de diamètre sont découpés.

### Exemple 5

Un lot d'implants contenant du miconazole à 50% et du PLGA 85:15 à 50% est préparé selon le procédé décrit ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 4.5-5.5 | 160 | 0,85 | 1500-200 |

Des implants de 9mm de longueur et 0,58mm de diamètre sont découpés.

### Exemple 6

Un lot d'implants contenant de l'amorolfine et du PLGA 85:15 est préparé selon le procédé décrit ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 2-10 | 160 | 1,5 | - |

Des implants de 10,90 mm de longueur et 0,58 mm de diamètre sont découpés.
Les analyses sont réalisées suivant le procédé décrit ci-dessus en B (5).

| Contenu API% m/m | Pureté actif % | Dose implant |
|---|---|---|
| 46,30 | 100 | 1,54 |

La libération *In Vitro* est réalisée tel que décrit ci-dessus en B (5). Les résultats sont représentés sur la Figure 3.

L'évaluation de l'activité antifongique *In Vitro* est réalisée tel que décrit ci-dessus en C.

### Exemple 7

Un lot d'implants contenant de la prèle à 30% et 70% PLGA 85:15 est préparé selon le procédé décrit ci-dessus en A (1).

Les conditions d'extrusion sont les suivantes :

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 10 à 150°C 5 min puis 2 à 180°C | 150-180 | 0,85 | 2000-3000 |

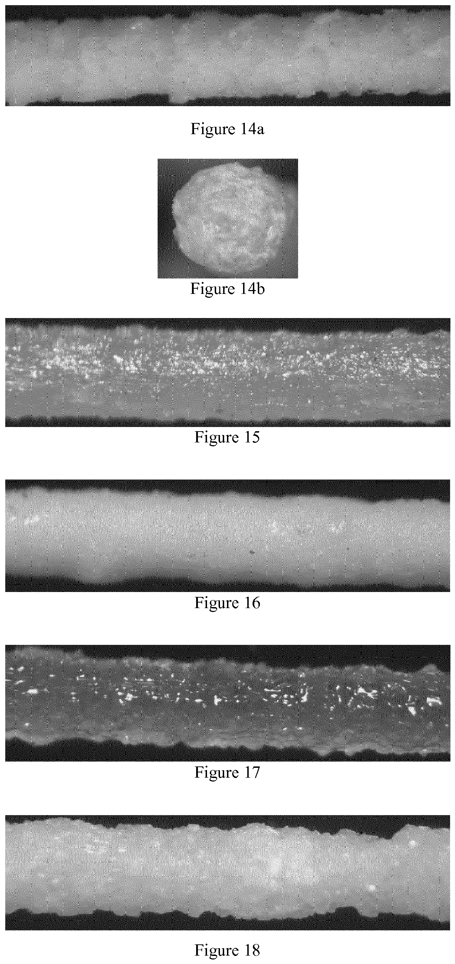

Des implants d'une longueur de 10mm et de 0,4-0,8mm de diamètre sont ensuite découpés. La Figure 15 représente une photo des implants réalisés dans l'Exemple 7 prise à la loupe binoculaire.

### Exemple 8

Un lot d'implants contenant de l'itraconazole et du PLGA 85:15 est préparé selon le procédé décrit ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 10 | 110-170 | 0,7 | 3000 |

Des implants d'une longueur de 6mm et de 0,7mm de diamètre sont ensuite découpés.
Les analyses sont réalisées suivant le procédé décrit ci-dessus en B (6).

| Contenu % m/m | Pureté actif % | Dose implant |
|---|---|---|
| 60,90 | 99,3 | 1,9 |

La Figure 16 représente une photo des implants réalisés dans l'Exemple 8 prise à la loupe binoculaire.

### Exemple 9

Un lot d'implants contenant 12% de lactate, du PLGA 85 :15 et de la Terbinafine est préparé selon le procédé décrit dans ci-dessus en A (1).

| Diamètre Vis (mm) | Vitesse extrusion (rpm) | Température d'extrusion (T°C) | Diamètre de buse (mm) | Pression en sortie extrusion (mPa) |
|---|---|---|---|---|
| 8 | 12 | 110-120 | 0,85 | 750 |

Des implants de 10mm de longueur et 0,8mm de diamètre sont découpés.

Les analyses sont réalisées suivant le procédé décrit ci-dessus en B (1) et B (2).

| Contenu % m/m | Pureté actif % | Dose implant mg |
|---|---|---|
| 58,20 | 99,53 | 2,15 |

Le release *In Vitro* est réalisé tel que décrit ci-dessus en B (2).

### Exemple 10

Un lot d'implants contenant 50% de PLGA 85:15 et 50% L-lactate est préparé selon le procédé décrit ci-dessus en A (1).

| Diamètre Vis(mm) | Vitesse extrusion (rpm) | Température d'extrusion (T°C) | Diamètre de buse (mm) | Pression en sortie extrusion (mPa) |
|---|---|---|---|---|
| 8 | 10-20 | 120 | 0,85 | 49 |

Des implants de 10mm de longueur et 0,8mm de diamètre sont découpés.

### Exemple 11

Un lot d'implants contenant 12% de PLGA 50:50, 28% de PLGA 85 :15 et 60% de Terbinafine est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 10 | 166-100 | 0,5-0,85 | 2000-3000 |

Des implants de 10mm de longueur et 0,6mm de diamètre sont découpés.

Les analyses sont réalisées suivant le procédé décrit ci-dessus en B (1) et B (2).

| Contenu % m/m | Pureté actif % | Dose implant mg |
|---|---|---|
| 59,60 | 99,53 | 1,65 |

### Exemple 12

Un lot d'implants contenant 100% de PLGA 85:15 est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 2-3 | 146-154 | 0.85 | - |

Des implants de 10mm de longueur et 0,80mm de diamètre sont découpés.

Un essai d'activité antifongique est réalisé tel que décrit ci-dessus en C avec deux souches : Candida Krusei et Candida Parapsilosis. Les résultats du halo d'inhibition sont donnés en millimètre autour de l'implant dans la table suivante :

| t | 10 jours | 14 jours | 20 jours | 28 jours |
|---|---|---|---|---|
| Candida Krusei | 4 mm | - | 3 mm | 3 mm |
| Candida Parapsilosis | 4 mm | 5 mm | 3 mm | 3 mm |

### Exemple 13

Un lot d'implants contenant 12% de PLA 202S, 28% de PLGA 85 :15 et 60% de Terbinafine est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 8 | 100 | 0,85 | 1600 |

Des implants de 10mm de longueur et 0,55mm de diamètre sont découpés.

Les analyses sont réalisées suivant le procédé décrit ci-dessus en B (1) et B (2).

| Contenu % m/m | Pureté actif % | Dose implant mg |
|---|---|---|
| 59,30 | 99,53 | 1,51 |

### Exemple 14

Un lot d'implants contenant 50% de PLA 202S et 50% de PLGA 85:15 est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 8 rpm | 100 | 0,85 | 1600 |

Des implants de 10mm de longueur et 0,55mm de diamètre sont découpés.

### Exemple 15

Un lot d'implants contenant 25% de chitosan et 75% de PLGA 85 :15 est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 10 | 150 | 0,85 | 1500 |

Des implants de 10mm de longueur et 0,60mm de diamètre sont découpés.

### Exemple 16

Un lot d'implants contenant 20% de chitosan, du PLGA 85:15 et de la Terbinafine est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 10 | 148 | 0,85 | 2000 |

Des implants de 10mm de longueur et 0,60mm de diamètre sont découpés.

Les analyses sont réalisées suivant le procédé décrit ci-dessus en B (1) et en B (2).

| Contenu % m/m | Pureté actif % | Dose implant mg |
|---|---|---|
| 8,80 | 99,63 | 0,33 |

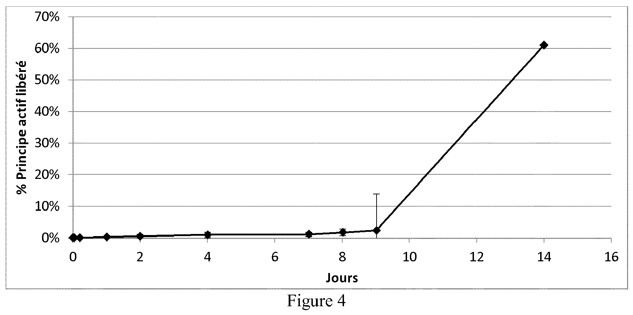

Le test de diffusion *In Vitro* est réalisé tel que décrit ci-dessus. Les résultats sont représentés sur la Figure 4.

### Exemple 17

Un lot d'implants contenant du Minoxidil et du PLGA 85:15 est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion (mPa) |
|---|---|---|---|---|
| 8 | 10 | 148 | 0,85 | 2000 |

Des implants de 8mm de longueur et 0,75mm de diamètre sont découpés.

### Exemple 18

Des lots d'implants contenant différentes concentrations de ciclopirox dans du PLGA 85:15 sont préparés selon le procédé décrit ci-dessus en en A (1).

| | Diamètre vis (mm) | Vitesse extrusion (rpm) | Température d'extrusion (T°C) | Diamètre de buse (mm) | Pression | Longueur et diamètre (mm) |
|---|---|---|---|---|---|---|
| (1) | 8 | 2 | 130 | 0,85 | N/A | 10 x 0,30mm |
| (2) | 8 | 12 | 102 | 0.85 | 150 bar | 10 x 0,40 mm |
| (3) | 8 | 10 | 110 | 0,70 | 75 bar | 10 x 0,60 mm |
| (4) | 8 | 10 | 130 | 1 | N/A | 9 x 0,65 mm |

Les analyses sont réalisées suivant le procédé décrit ci-dessus en B (3) et en B (4).

| | Contenu % m/m | Pureté actif % | Dose implant mg |
|---|---|---|---|
| (1) | 15,50 | 97,60 | 0,15 |
| (2) | 34,80 | 98,10 | 0,51 |
| (3) | 59,90 | 98,40 | 2,50 |
| (4) | 65,80 | 98,20 | 1,80 |

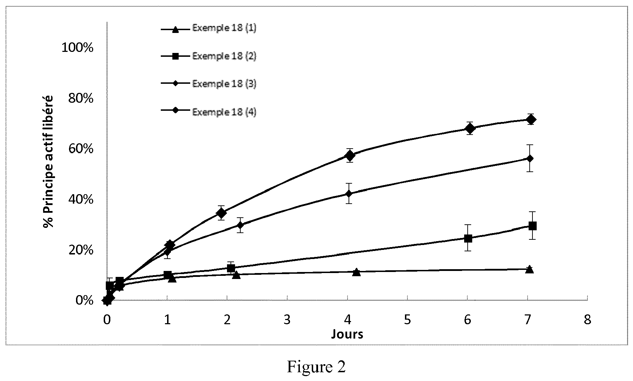

Le test de libération *In Vitro* est réalisé tel que décrit dans ci-dessus en B (4). Les résultats sont représentés sur la Figure 2.

L'évaluation de l'activité antifongique *In Vitro* est réalisée tel que décrit ci-dessus en C.

Le test *In Vivo* est réalisé tel que décrit ci-dessus en E.

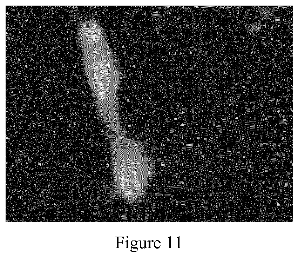

La Figure 11 représente la photo d'un implant de composition décrite ci-dessus en 18 (3) après 84 jours d'implantation.
Les résultats de l'implantation *In Vivo* sont résumés sur la Figure 13.

La stabilité du lot (2) est déterminée telle que décrit ci-dessus en D à 5°C, 25°C/60% d'humidité relative et 40°C/75% d'humidité relative, à 1 mois, 3 mois et 6 mois. Les résultats de contenu en ciclopirox, de pureté et de dose d'actif obtenus sont les suivants :

| **Lot** | **Paramètre** | **Conditions** | **T0** | **T1M** | **T3M** | **T6M** |
|---|---|---|---|---|---|---|
| **Exemple 18(2)** | **Contenu en API (%m/m)** | 5°C | 34,80 | 33.9 | 35.0 | 36.8 |
| | | 25°C / 60%RH | | 34.5 | 33.8 | 35.7 |
| | | 40°C / 75%RH | | 33.0 | NA | NA |
| | **Pureté (%, w/w)** | 5°C | 98,1 | 98.6 | 98.3 | 98.6 |
| | | 25°C / 60%RH | | 98.6 | 98.3 | 98.4 |
| | | 40°C / 75%RH | | 98.7 | NA | NA |
| | **Dose actif (mg)** | 5°C | 0.51 | 0.46 | 0.52 | 0.54 |
| | | 25°C / 60%RH | | 0.53 | 0.49 | 0.51 |
| | | 40°C / 75%RH | | 0.51 | NA | NA |

La stabilité du lot (3) est déterminée selon le procédé décrit ci-dessus en D à 5°C, 25°C/60% d'humidité relative et 40°C/75% d'humidité relative, à 1 mois, 3 mois et 6 mois. Les résultats de contenu en ciclopirox, de pureté et de dose d'actif obtenus sont les suivants :

| **Lot** | **Paramètre** | **Conditions** | **T0** | **T1M** | **T3M** | **T6M** |
|---|---|---|---|---|---|---|
| **Exemple 18(3)** | **Contenu en API (%m/m)** | 5°C | 59,90 | 58.6 | 57.5 | 60.2 |
| | | 25°C / 60%RH | | 56.8 | 61.3 | 59.4 |
| | | 40°C / 75%RH | | 57.6 | NA | NA |
| | **Pureté (%, w/w)** | 5°C | 98,40 | 98.4 | 98.4 | 98.8 |
| | | 25°C / 60%RH | | 98.3 | 98.5 | 98.7 |
| | | 40°C / 75%RH | | 98.3 | NA | NA |
| | **Dose actif (mg)** | 5°C | 2,50 | 2.18 | 2.39 | 2.28 |
| | | 25°C / 60%RH | | 2.23 | 2.37 | 2.40 |
| | | 40°C / 75%RH | | 2.22 | NA | NA |

### Exemple 19

Un lot d'implants contenant du Ciclopirox et du PLGA 85 :15 est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion |
|---|---|---|---|---|
| 8 | 10-17 | 130-126 | 1 | 600-2300 |

Des implants de 7mm de longueur et 1mm de diamètre sont découpés.
Les analyses sont réalisées suivant le procédé décrit ci-dessus en B (3) et B (4).

| Contenu % m/m | Pureté actif % | Dose implant mg |
|---|---|---|
| 55,00 | 98,9 | 1,98 |

L'évaluation de l'activité antifongique In Vitro est réalisée selon le procédé décrit ci-dessus en C. La Figure 7 représente la photo d'une plaque d'agar après implantation d'un composé selon l'Exemple 19 à 35°C dans C. Parapsilosis.

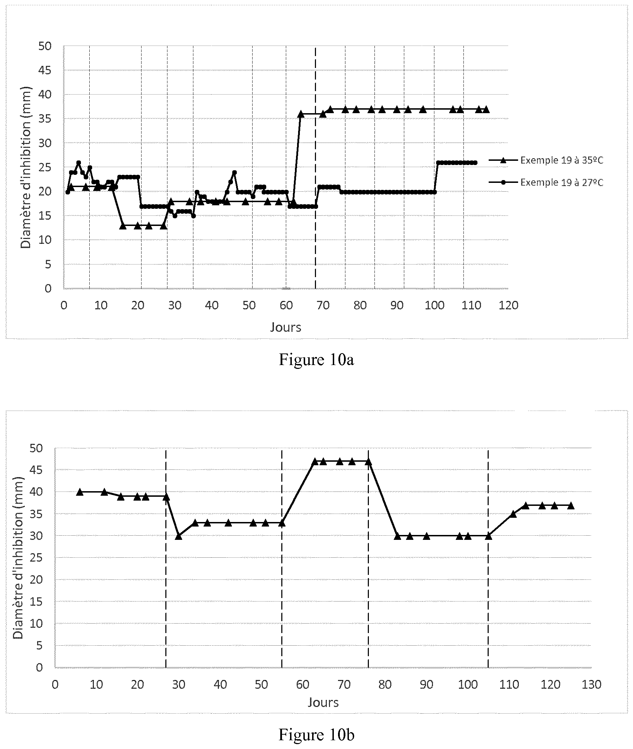

Les résultats sont résumés sur les Figures 10a et 10b.

### Exemple 20

Un mélange 50% PLGA 85 :15 et 50% PLA 202S est préparé selon le procédé décrit ci-dessus en A (2). Les poinçons sont placés en configuration de 1,4 mm pour une épaisseur de 4. Le lot comporte 19 implants.

Le procédé utilise une force de compression de 220-250N pour une force d'éjection de 20-25N.

Les implants obtenus ont les caractéristiques suivantes :

| Masse (mg) | Longueur (mm) | Diamètre (mm) | Densité (g/mL) | Volume (µL) |
|---|---|---|---|---|
| 1,05 | 1,61 | 0.91 | 1,11 | 1,05 |

### Exemple 21

Un filament contenant 100% de PLGA 85 :15 est préparé selon le procédé d'impression décrit ci-dessus en A (3).

| Diamètre vis (mm) | Vitesse extrusion (rpm) | Température d'extrusion (T°C) | Diamètre de buse (mm) | Pression en sortie extrusion |
|---|---|---|---|---|
| 8 | 12 | 135-141 | 2,5 | 60 MPa |

Ce filament de diamètre 1.55-1.75mm est ensuite utilisé pour être imprimé en 3D tel que décrit ci-dessus en A (3). Les conditions d'impression sont les suivantes :

| | |
|---|---|
| - Température d'extrusion : | 190°C |
| - Température verre (lit) : | 50°C |
| - Alimentation: | 60% de l'alimentation maximale |
| - Vitesse : | 80% de la vitesse maximale |

Les implants sont imprimés par lot de 10, et mesurent 10 mm pour 0,4 mm de diamètre.

### Exemple 22

Un lot d'implants contenant du Ciclopirox et du PLA 202H est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion |
|---|---|---|---|---|
| 8 | 6-10 | 80 | 1 | N/A |

Des implants de 10mm de longueur et 0,8mm de diamètre sont découpés.
Les analyses sont réalisées suivant le procédé décrit ci-dessus en B (3) et B (4).

| Contenu % m/m | Pureté actif % | Dose implant mg |
|---|---|---|
| 41,90 | 99,95 | 2,37 |

### Exemple 23

Un lot d'implants contenant approximativement 30% (w/w) d'Arginine et du PLGA 85 :15 est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion |
|---|---|---|---|---|
| 8 | 8 | 150 | 1 | 1500 MPa |

Des implants de 10mm de longueur et 0,9mm de diamètre sont découpés.

### Exemple 23

Un lot d'implants contenant approximativement 30% (w/w) de Ciclopirox et 30% (w/w) de Terbinafine pour obtenir au total un mélange avec du PLGA 85 :15 ayant un contenu en principe actif à 60% (w/w) est préparé selon le procédé décrit ci-dessus en A (1).

| Diamètre vis (mm) | Vitesse extrusion (rpm) | Température d'extrusion (T°C) | Diamètre de buse (mm) | Pression en sortie extrusion |
|---|---|---|---|---|
| 8 | 10-12 | 130-140 | 1 | 90-150 bar |

Des implants de 10mm de longueur et 0,8mm de diamètre sont découpés.

## Revendications

1. Composition pour son utilisation dans le traitement de maladies de l'ongle et/ou pour accélérer la croissance de l'ongle **caractérisée en ce qu'**elle comprend un ou plusieurs polymères solides biodégradables à libération prolongée, et éventuellement un ou plusieurs principes actifs et **en ce qu'**elle se présente sous la forme d'un dispositif destiné à être administré par voie sous-cutanée à une distance supérieure à 1cm du bord de l'ongle de préférence dans le doigt à l'exception de la dernière phalange.

2. Composition comprenant un ou plusieurs polymères solides biodégradables à libération prolongée, et éventuellement un ou plusieurs principes actifs pour son utilisation dans le traitement de maladies de l'ongle et/ou pour accélérer la croissance de l'ongle par administration par voie sous-cutanée à une distance supérieure à 1cm du bord de l'ongle de préférence dans le doigt à l'exception de la dernière phalange.

3. Composition selon la revendication 1 ou 2, comprenant un ou plusieurs principes actifs choisis parmi :
- Les antis infectieux choisis parmi :
les agents antifongiques,
les antibiotiques
et les antiparasitaires
- Les anti-inflammatoires choisis parmi
les anti-inflammatoires stéroïdiens
les anti-inflammatoires non stéroïdiens et
les inhibiteurs de Janus Kinase (JAK),
- Les ciclosporines,
- la vitamine D ou les dérivés de la vitamine D,
- Les immunosuppresseurs,
- Les acides aminés, de préférence l'Arginine,
- Les principes actifs accélérant la croissance de l'ongle choisis de préférence parmi
les prostaglandines.
le Chitosan,
l'Acide valproïque
l'Echisetum arvense (la prêle), l'Acide hyaluronique, la Biotine, l'Hormone de croissance (GH), le Minoxidil, la Finasteride.
- Les principes actifs favorisant la circulation sanguine choisis parmi
les vasodilatateurs et
les anti agrégants plaquettaires,
- Les anesthésiques locaux,
pour son utilisation dans le traitement de maladies de l'ongle par administration par voie sous-cutanée à une distance supérieure à 1cm du bord de l'ongle de préférence dans le doigt à l'exception de la dernière phalange.

4. Composition selon l'une des revendications précédentes pour son utilisation dans le traitement de maladies de l'ongle, de préférence l'onychomycose **caractérisée en ce que** la composition comprend un ou plusieurs principes actifs choisis parmi :
- Les agents antifongiques,
- Les anti-inflammatoires stéroïdiens
- Les anti-inflammatoires non stéroïdiens et les inhibiteurs de Janus Kinase (JAK),
- Les principes actifs accélérant la croissance de l'ongle choisis de préférence parmi les-prostaglandines.-l'Echisetum arvense (la prêle), le Chitosan, l'Acide hyaluronique, la Biotine, l'Acide valproïque, l'Hormone de croissance (GH), le Minoxidil et/ou le Finasteride,
- Les acides aminés, de préférence l'Arginine,
- Les anesthésiques locaux
- Les vasodilatateurs,
- Les antibiotiques
- Les antiparasitaires

5. Composition selon l'une des revendications 1 à 3 pour son utilisation dans le traitement de maladies de l'ongle, **caractérisée en ce que** la maladie de l'ongle est le psoriasis accompagné ou non d'un phénomène inflammatoire et **en ce que** la composition comprend un ou plusieurs principes actifs choisis parmi :
- Les anti-inflammatoires stéroïdiens
- Les anti-inflammatoires non stéroïdiens
- les inhibiteurs de Janus Kinase (JAK),
- Les ciclosporines,
- Les acides aminés, de préférence l'Arginine,
- La vitamine D ou des dérivés de la vitamine D,
- des immunosuppresseurs,
- des rétinoïdes,
- des composés tels que l'Anthralin (ou dithranol) et l'Urée.
- un ou plusieurs principes actifs accélérant la croissance de l'ongle choisis de préférence parmi les prostaglandines. l'Echisetum arvense (la prêle), le Chitosan, l'Acide hyaluronique, la Biotine, l'Acide valproïque, l'Hormone de croissance (GH), le Minoxidil et/ou le Finasteride.
- Les vasodilatateurs,
- Les anesthésiques locaux

6. Composition selon l'une des revendications 1 à 3 pour son utilisation pour accélérer la croissance de l'ongle **caractérisée en ce qu'**elle comprend un ou plusieurs principes actifs choisis parmi :
- les ciclosporines
- les prostaglandines
- l'Echisetum arvense (la prêle), le Chitosan, l'Acide hyaluronique, l'Arginine, la Biotine, l'Acide valproïque, l'Hormone de croissance (GH), le Minoxidil et/ou le Finasteride.
- Les vasodilatateurs
- l'acide valproïque.

7. Composition selon l'une des revendications précédentes pour son utilisation dans le traitement de maladies de l'ongle et/ou pour accélérer la croissance de l'ongle **caractérisée en ce qu'**elle se présente sous la forme d'un implant présentant éventuellement une extrémité aiguisée, d'un tube cylindrique ou d'un fil et **en ce que** la composition est administrée dans le doigt à l'exception de la dernière phalange.

8. Composition selon l'une des revendications précédentes **caractérisée en ce que** le polymère biocompatible à libération prolongée, de préférence biodégradable ou bio-résorbable, de préférence un polyester,et encore plus preferentiellement un copolymère acide lactique-acide glycolique (PLGA) ou un polymère de l'acide lactique (PLA) et **en ce que** la charge en principe actif est comprise entre 0 et 98%, de préférence inférieure ou égale à 85% et encore plus préférentiellement comprise entre 20 et 65% en poids.

9. Composition selon l'une des revendications précédentes **caractérisée en ce qu'**elle comprend entre 0,04 et 6.5mg de ciclopirox ou de chlorhydrate de terbinafine et du PLGA.

10. Composition, selon l'une des revendications précédentes de préférence sous forme d'implant pour son utilisation dans le traitement des mycoses de l'ongle **caractérisée en ce qu'**elle comprend un ou plusieurs polymères solides biodégradables à libération prolongée, et éventuellement un ou plusieurs antifongiques et **en ce qu'**elle se présente sous la forme d'un implant ayant une longueur maximale de 25 mm de préférence entre 4 et 15 mm et un diamètre compris entre 0,1 et 1 mm de préférence entre 0,3 et 0,8 mm, implant destiné à être administré par voie sous-cutanée à une distance supérieure à 1cm du bord de l'ongle dans le doigt à l'exception de la dernière phalange.

11. Composition selon l'une des revendications précédentes pour son utilisation dans le traitement de maladies de l'ongle et/ou pour accélérer la croissance de l'ongle **caractérisée en ce qu'**elle se présente sous la forme d'un implant, d'un tube cylindrique ou d'un fil, présentant éventuellement une extrémité aiguisée, et **en ce que** la composition est administrée dans le doigt à l'exception de la dernière phalange, dans la première ou la deuxième phalange des doigts, de préférence la deuxième phalange et dans la première phalange du pouce ou du gros orteil, **en ce qu'**une ou plusieurs compositions, de préférence de une à huit compositions, plus préférentiellement de une à six compositions sont administrées simultanément dans le même doigt et **en ce que** le traitement est continu, de préférence pendant une durée minimale de 48 semaines lorsque la totalité de l'ongle est affecté, une durée minimale de 24 semaine lorsque moins de la moitié de l'ongle est affecté, une durée minimale de 12 semaines lorsque moins du quart de l'ongle est affecté, ou encore d'une durée minimale de 6 semaines lorsque moins de 10% est affecté.

12. Composition non solide pour son utilisation dans le traitement de maladies de l'ongle et/ou pour accélérer la croissance de l'ongle comprenant un ou plusieurs polymères biodégradables à libération prolongée, et éventuellement un ou plusieurs principes actifs de préférence un ou plusieurs antifongiques **caractérisée en ce qu'**elle se présente sous la forme d'un dispositif destinée à être administrée par insertion dans l'espace entre le lit et la plaque de l'ongle et **en ce qu'**elle est éventuellement, utilisée de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions selon l'une quelconque des revendications 1 à 11.

13. Vernis à ongles à action antimycotique comprenant un ou plusieurs antifongiques choisis de préférence parmi le fluconazole, le ketoconazole, le miconazole, la terbinafine, le tolnaftate, l'amorolfïne, le ciclopirox, ou le miconazole, pour son utilisation dans le traitement de maladies de l'ongle par application sur les ongles de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions selon l'une quelconque des revendications 1 à 11 et/ou avec l'administration par insertion dans l'espace entre le lit et la plaque de l'ongle d'une ou plusieurs compositions selon la revendication 12.

14. Composition pharmaceutique antifongique comprenant un ou plusieurs antifongiques choisis de préférence parmi les dérivés azotés tels que le kétoconazole, le fluconazole, l'isavuconazole, l'traconazole, le posaconazole ou le voriconazole; les échinocandines telles que l'anidulafungine, la caspofungine, la micafungine, la flucytosine, la griséofulvine et la terbinafine; l'amphotéricine B pour son utilisation dans le traitement de maladies de l'ongle par administration par voie orale ou injectable de façon simultanée ou étalée dans le temps avec l'administration par voie sous-cutanée à une distance supérieure à 1cm du bord de l'ongle de préférence dans le doigt à l'exception de la dernière phalange d'une ou plusieurs compositions selon l'une quelconque des revendications 1 à 11 et/ou avec l'administration par insertion dans l'espace entre le lit et la plaque de l'ongle d'une ou plusieurs compositions selon la revendication 12 et/ou avec l'application sur les ongles d'un vernis à ongle à action antifongique selon la revendication 13.

15. Composition selon l'une des revendications 1 à 11 **caractérisée en ce qu'**elle se présente de préférence sous la forme d'un implant présentant éventuellement une extrémité aiguisée, d'un tube cylindrique ou d'un fil et **en ce que** la composition est administrée dans le doigt à l'exception de la dernière phalange pour son utilisation dans le traitement de maladies de l'ongle par administration de façon simultanée ou étalée dans le temps avec :
- l'administration par insertion dans l'espace entre le lit et la plaque de l'ongle d'une composition non solide comprenant un ou plusieurs polymères biodégradables à libération prolongée, et éventuellement un ou plusieurs principes actifs de préférence un ou plusieurs antifongiques
et/ou
- l'application sur les ongles d'un vernis à ongles à action antimycotique comprenant un ou plusieurs antifongiques choisis de préférence parmi le fluconazole, le ketoconazole, le miconazole, la terbinafine, le tolnaftate, l'amorolfine, le ciclopirox, ou le miconazole,
et/ou
- l'administration par voie orale ou injectable d'une composition pharmaceutique antifongique comprenant un ou plusieurs antifongiques choisis de préférence parmi les dérivés azotés tels que le kétoconazole, le fluconazole, l'isavuconazole, l'traconazole, le posaconazole ou le voriconazole; les échinocandines telles que l'anidulafungine, la caspofungine, la micafungine, la flucytosine, la griséofulvine et la terbinafine; l'amphotéricine B
et/ou
- un traitement par laser provoquant la destruction du champignon par effet thermique photobiologique.
